# EUROPÄISCHE PATENTANMELDUNG

(11) **EP 4 830 741 A2**
(43) Veröffentlichungstag der Anmeldung: **28.10.2026**
(21) Anmeldenummer: 26204842.4
(22) Anmeldetag: 12.12.2024
(51) Int. Cl.: A61L 31/16

(54) **VERFAHREN, VORRICHTUNG UND KIT ZUR HERSTELLUNG EINER PHARMAZEUTISCH AKTIVEN BESCHICHTUNG**

(62) Dokumentnummer(n) der früheren Anmeldung(en) nach Art. 76 EPÜ: 24219291.2 / 4 759 337
(71) Anmelder: Heraeus Medical GmbH, 61273 Wehrheim (DE)
(72) Erfinder: Dr. Vogt, Sebastian, 61273 Wehrheim (DE); Dr. Kluge, Thomas, 61273 Wehrheim (DE)
(74) Vertreter: Heraeus IP

(57) **Zusammenfassung**

Beschrieben wird ein Verfahren zur Beschichtung von Implantaten mit einer Polymerschicht, die mindestens einen pharmazeutischen Wirkstoff enthält. Beschrieben wird ferner ein Kit-of-parts, welches zur Durchführung des Verfahrens geeignet ist, und eine entsprechende Vorrichtung, die aus dem Kit-of-parts erhalten wird.

## Beschreibung

Die vorliegende Erfindung betrifft ein Verfahren zur Beschichtung von Implantaten mit einer Polymerschicht, die mindestens einen pharmazeutischen Wirkstoff enthält. Weiterer Gegenstand der Erfindung ist ein Kit-of-parts, welches zur Durchführung des erfindungsgemäßen Verfahrens geeignet ist, und eine entsprechende Vorrichtung, die aus dem Kit-of-parts erhalten wird.

### HINTERGRUND DER ERFINDUNG UND STAND DER TECHNIK

Gegenstand der vorliegenden Erfindung ist ein Verfahren zur Beschichtung von Implantaten mit einer Polymerschicht, die mindestens einen pharmazeutischen Wirkstoff enthält. Das Verfahren ist insbesondere zur intraoperativen Beschichtung, also der Beschichtung in unmittelbarer zeitlicher Nähe zu einer Operation, von in der Unfallchirurgie und Orthopädie üblichen Implantaten, wie intramedulläre Nägel und Osteosyntheseplatten, mit Polymeren geeignet, in denen pharmazeutische Wirkstoffe, bevorzugt Antiinfektiva, dispergiert sind. Die beschichteten Implantate sollen insbesondere bei septischen Revisionen in der Unfallchirurgie und Orthopädie Anwendung finden. Die beschichteten Implantate stellen nach erfolgter Implantation in den humanen Organismus lokale Wirkstofffreisetzungssysteme dar. Wässrige Körperflüssigkeiten, wie Blut und Wundsekret, können die pharmazeutischen Wirkstoffe aus der Polymerschicht herauslösen und in die Umgebung der Implantate freisetzen. Die Oberflächen der Implantate können durch die freigesetzten pharmazeutischen Wirkstoffe beispielsweise vor einer mikrobiellen Besiedlung temporär geschützt werden.

Intramedulläre Nägel und Osteosyntheseplatten sind Stand der Technik und werden weltweit in großem Umfang erfolgreich zur Behandlung von Knochenfrakturen in der Unfallchirurgie verwendet. Leider kommt es nach erfolgter Versorgung der Frakturen mitunter zu Infektionen des umliegenden Knochen- und Weichgewebes mit Mikroorganismen, insbesondere mit Bakterien, und dabei häufig mit Hautkeimen, wie *Staphyloccus aureus* und *Staphylococus epdidermidis*. Bei dem Auftreten solcher Infektionen ist eine chirurgische Sanierung der infizierten Gewebeareale unbedingt notwendig, bei der infizierte Gewebeareale abgetragen werden. Weiterhin ist eine systemische Antibiose nach erfolgtem Debridement zur Keimreduktion üblich. Wünschenswert wäre es, wenn nach der chirurgischen Sanierung eine mechanische Stabilisierung des frakturierten Knochengewebes durch intramedulläre Nägel oder durch Osteosyntheseplatten weiterhin möglich wäre und diese gleichzeitig lokal Antiinfektiva abgegeben können, um im debridierten Gewebe verbliebene mikrobielle Keime lokal zu unterdrücken.

Intramedulläre Nägel mit einer Antibiotika enthaltenden Polymethylmethacrylat-Knochenzementbeschichtung sind seit langem bekannt und zeigen klinische Ergebnisse (N. Walter, M. Rupp, J. Krueckel, Volker Alt: Individual and commercially available antimicrobial coatings for intramedullary nails für the treatment of infected long bone non-unions: a systematic review. Injury 53S3 (2022) S75-S80.). Die in der Polymethylmethacrylat-Knochenzementschicht enthaltenen Antibiotika-Partikel werden nach der Implantation der beschichten intramedullären Nägel durch wässrige Körperflüssigkeiten, wie Wundsekret und Blut, aus der Knochenzementschicht herausgelöst und führen zu einer lokal hohen Antibiotika-Konzentration an der Oberfläche der beschichteten intramedullären Nägel. Diese hohen lokalen Antibiotika-Konzentrationen reduzieren in Kombination mit der systemischen Antibiose die nach dem Debridement noch verbliebenen mikrobiellen Keime.

Besonders interessant sind dabei intraoperativ beschichtete intramedulläre Nägel. Bei der intraoperativen Beschichtung ist es möglich, exakt auf die vorliegenden Keime abgestimmte Antiinfektiva in die Beschichtung, beispielsweise in die Polymethylmethacrylat-Knochenzement, einzubringen. Bisher erfolgt die intraoperative Beschichtung so, dass Antiinfektiva in das Zementpulver eingemischt werden, das so modifizierte Zementpulver mit einer Monomerlösung vermischt und der dann gebildete Zementteig um den zu beschichtenden Nagel manuell geformt wird. Dabei ist es schwierig, eine gleichmäßige Schichtdicke über die gesamte Länge des intramedullären Nagels zu erzielen. Es kann daher passieren, dass der beschichtete Nagel zumindest teilweise einen zu großen Querschnitt aufweist und nicht mehr in den zuvor debridierten Knochen implantiert werden kann.

Eigene praktische Erfahrungen zeigen, dass dem Polymethylmethacrylat-Knochenzement relativ geringen Mengen an Antiinfektiva zugesetzt werden können, weil der Zementteig bei höheren Gehalten an Antiinfektiva zu viskos wird, um damit intramedulläre Nägel beschichten zu können. Des Weiteren wird die Haftung des Zements auf Metalloberflächen mit steigenden Gehalten an Antiinfektiva beeinträchtigt.

Wünschenswert ist es, wenn eine Beschichtung mit einer geringen Schichtdicke und gleichzeitig einem hohen Anteil an Antiinfektiva möglich wäre. Damit würde es keine Probleme bezüglich der radialen Ausdehnung der intramedullären Nägel geben und gleichzeitig wäre eine hohe lokale Freisetzung von Antiinfektiva möglich.

Neben der Beschichtung mit antiinfektiv ausgerüsteten Polymethylmethacrylat-Knochenzement ist auch eine direkte Beschichtung von Implantaten mit Antibiotika und mit Antibiotika, die in Polymeren suspendiert oder gelöst sind, möglich.

In US 2007/0134287 A wird eine antibiotische Beschichtungslösung beschrieben, die aus einem leicht verdampfenden Lösungsmittel und einem darin gelösten Antibiotikum besteht.

In US 11,517,650 B wird eine ähnliche Beschichtungslösung beschrieben, bei der Antibiotika in einem schnell verdampfenden Lösungsmittel gelöst werden, wobei zur Beschleunigung des Lösungsprozesses Energie durch eine Einstrahlung von Ultraschall zugeführt wird. Die Lösung wird dann zur antibiotischen Beschichtung eingesetzt.

In EP 1 243 259 B wird eine Beschichtung offenbart, die aus Polymethylmethacrylat und einem hydrophilen Polymer, wie einem Polyether, besteht, in der ein Antibiotikum suspendiert ist. Der Nachteil der offenbarten Copolymere ist, dass die Polymerbeschichtung, die gemäß EP 1 243 259 B erhalten wird, verhältnismäßig dick ausfällt. Zusätzlich ist es erforderlich, der Beschichtung den hydrophilen Polyether zuzusetzen, um eine ausreichende Freisetzung des Antibiotikums zu erreichen. Der hydrophile Polyether ist wasserlöslich und dient als Porenbildner.

Ein ähnliches Konzept wird in EP 1 112 095 B beschrieben. Hierbei wird eine Schicht aus D,L-Polylactid vorgeschlagen, in der ein Antibiotikum suspendiert ist. Die Beschichtung erfolgt in der Weise, dass zuerst das Polylactid in einem schnell verdampfenden Lösungsmittel gelöst wird und dann Antibiotikumpartikel in dieser Lösung suspendiert werden. Diese Suspension wird auf Implantatoberflächen aufgetragen, wobei das Lösungsmittel verdunstet und ein D,L-Polylactidfilm zurückbleibt. In dem D,L-Polylactidfilm sind die Antibiotikumpartikel fixiert und können durch Einwirkung wässriger Körperflüssigkeiten, wie Wundsekret und Blut, herausgelöst werden. Der D,L-Polylactidfilm ist durch Einwirkung von Wasser oder wässrigen Lösungen hydrolysierbar und zersetzt sich zu wasserlöslichen Folgeprodukten.

Die aus dem Stand der Technik beschriebenen Verfahren zur Beschichtung sind in ihrer Anwendung in der Regel komplex durchzuführen und zeitintensiv. Darüber hinaus ermöglichen die Verfahren des Standes der Technik nur begrenzt eine Variation der pharmazeutischen Wirkstoffe und eine Auswahl geeigneter pharmazeutischen Wirkstoffe unmittelbar vor oder während einer Operation ist nicht möglich. Hinzukommend sind die im Stand der Technik beschriebenen Verfahren dahingehend nachteilig, dass in der Regel verhältnismäßig dicke Beschichtungen resultieren und ungeeignete Bestandteile verwendet werden müssen, um eine Freisetzung aus der Polymermatrix zu ermöglichen.

### AUFGABE DER ERFINDUNG

Die Aufgabe der vorliegenden Erfindung besteht in der Entwicklung eines einfachen, zeitsparenden Verfahrens zur Beschichtung von Gegenständen mit einer pharmazeutisch aktiven Polymerschicht, die mindestens einen pharmazeutischen Wirkstoff enthält. Das Beschichtungsverfahren soll so gestaltet sein, dass das OP-Personal beliebige pharmazeutische Wirkstoffe in einfachster Weise in die Beschichtung einbringen kann. Die Polymerschicht soll aus einem biokompatiblen Polymer gebildet werden, dass nicht hydrolytisch oder enzymatisch im humanen Organismus degradiert wird. Weiterhin soll die Polymerschicht mit einfachsten Mittel unter OP-Bedingungen auf Oberflächen, wie von Implantaten und Osteosyntheseplatten, aufgebracht werden können. Die Polymerschicht soll sich in Gegenwart von Wasser bei 37 °C innerhalb von 7 Tagen nicht von der Oberfläche ablösen. Eine Gesundheitsgefährdung der OP-Personals während des Beschichtungsvorgangs durch giftige Lösungsmittel soll vermieden werden. Weiterhin soll es mit dem Verfahren möglich sein, Polymerschichten mit einem Mindestanteil von 10 Gew.-% Wirkstoff herzustellen. Die Polymerschicht soll außerdem nicht durch Einwirkung von wässriger Körperflüssigkeit erweichen und eine geringe, jedoch konstant zu erzielende Schichtdicke aufweisen. Schließlich soll die erfindungsgemäß herzustellende polymere Beschichtung eine gute Wasser- und Temperaturbeständigkeit aufweisen.

Zur Applikation der Polymerschicht soll ein Kit-of-parts mit Bestandteilen, die zum Herstellen und Applizieren einer mit Wirkstoffen ausgerüsteten Beschichtungslösung geeignet sind, bereitgestellt werden.

Eine weitere Aufgabe ist die Bereitstellung einer Vorrichtung, die ausgehend von dem Kit-of-parts erhalten wird.

Die Aufgaben der vorliegenden Erfindung werden mit den Maßnahmen gemäß den Ansprüchen 1 bis 15 gelöst.

### ZUSAMMENFASSUNG DER ERFINDUNG

Die vorliegenden Aufgaben werden zunächst durch ein Verfahren zur Herstellung einer pharmazeutisch aktiven Polymerbeschichtung gelöst.

Das erfindungsgemäße Verfahren ist dann durch mindestens die nachfolgenden Verfahrensschritte gekennzeichnet:
a) Auflösen eines nicht hydrolysierbaren Polymers oder Copolymers, welches durch eine zahlenmittlere Molmasse von größer als 200000 g/mol, weiter bevorzugt eine zahlenmittlere Molmasse von größer als 400000 g/mol, weiter bevorzugt eine zahlenmittlere Molmasse von größer als 600000 g/mol, bestimmt mittels Gelpermeations-Chromatografie gekennzeichnet ist, in einem Lösungsmittel oder Lösungsmittelgemisch, welches durch einen Dampfdruck von mindestens 95 hPa bei 20 °C und Normaldruck gekennzeichnet ist, unter Bildung einer Polymerlösung 1;
b) Auflösen oder Suspendieren von mindestens einem pharmazeutischen Wirkstoff in der Polymerlösung 1 unter Bildung einer Polymerlösung 2;
c) Auftragen der Polymerlösung 2 auf die Oberfläche eines zu beschichtenden Gegenstandes; und
d) Verdunsten des Lösungsmittels oder Lösungsmittelgemischs, sodass auf der Oberfläche des zu beschichtenden Gegenstandes eine Polymerschicht zurückbleibt, in welcher der mindestens eine pharmazeutische Wirkstoff dispergiert ist.
dadurch gekennzeichnet, dass die Polymerschicht keine Porenbildner, insbesondere keine in wässrigen Lösungen löslichen Porenbildner aufweist.

Daneben betrifft die vorliegende Erfindung auch ein Kit-of-parts zur Ausführung des erfindungsgemäßen Verfahrens, umfassend die folgenden Bestandteile:
i) ein lösemittelbeständiger Behälter, der eine Öffnung aufweist;
ii) ein lösemittelbeständiges Verschlusselement, welches zum Verschließen des Behälters geeignet ist;
iii) eine Auftragehilfe;
iv) eine Polymerlösung 1, wie im erfindungsgemäßen Verfahren definiert;
v) gegebenenfalls ein oder mehrere Mischkörper; und
vi) gegebenenfalls ein oder mehrere pharmazeutische Wirkstoffe in separaten Verpackungsmitteln.

Vom Schutzbereich der vorliegenden Erfindung abgedeckt ist auch eine Vorrichtung zur Ausführung des erfindungsgemäßen Verfahrens, die mittels des medizinischen Kit-of-parts erhalten wird oder unabhängig hiervon bereitgestellt werden kann.

Weiterhin ist vom Umfang der vorliegenden Erfindung auch ein medizinisches Implantat abgedeckt, welches mit einer Polymerbeschichtung, erhältlich durch das erfindungsgemäße Verfahren, versehen ist. Auch die Verwendung des medizinischen Implantats zur Prävention von Infektionen nach einem operativen Eingriff wird vorliegend beansprucht.

Die vorliegende Erfindung wird nachfolgend im Detail beschrieben.

### DETAILLIERTE BESCHREIBUNG DER ERFINDUNG

### I. Definitionen

Der Begriff *Polymerlösung 1* bezeichnet im Sinne der vorliegenden Erfindung eine Lösung mindestens eines Polymers oder Copolymers, in welcher das Polymer oder Copolymer eine zahlenmittlere Molmasse von größer als 200000 g/mol, weiter bevorzugt eine zahlenmittlere Molmasse von größer als 400000 g/mol, weiter bevorzugt eine zahlenmittlere Molmasse von größer als 600000 g/mol, bestimmt mittels Gelpermeations-Chromatografie, aufweist und in einem Lösungsmittel oder Lösungsmittelgemisch mit einem Dampfdruck von mindestens 95 hPa bei 20 °C und Normaldruck, also bei 1013,25 hPa, vorliegt.

Der Begriff *Polymerlösung 2* bezeichnet im Sinne der vorliegenden Erfindung eine Lösung und/oder Suspension, bei der ein pharmazeutischer Wirkstoff in der Polymerlösung 1 gelöst oder suspendiert vorliegt.

Der Begriff *Auftragehilfe* im Sinne der vorliegenden Erfindung bezeichnet jedes mögliche Element, welches dazu geeignet ist, eine Lösung oder Suspension bevorzugt gleichmäßig auf eine Oberfläche zu verteilen. Es sind insbesondere streichende Elemente, sprühende Elemente oder auch Becken zum Eintauchen umfasst.

Der Begriff *Mischkörper* im Sinne der vorliegenden Erfindung bezeichnet ein Element, welches dazu geeignet ist, eine Lösung oder eine Suspension insbesondere auf mechanische Weise durchzumischen. Es sind insbesondere Mischstäbe wie Rührstäbe, aber auch Mischelemente wie Kugeln oder Rührfischchen von dem Begriff Mischkörper umfasst.

Wenn im Sinne der vorliegenden Erfindung von explizit bezeichneten Zahlenwerten in einem Bereich von X bis Y oder von mindestens X bis mindestens Y oder von größer als X bis größer als Y etc. die Rede ist, sind damit insbesondere auch alle implizit dazwischen liegenden Werte erfasst, die durch die Angabe der Nullstellen suggeriert werden. Liegt ein Wert also zwischen 1 und 10 sind damit insbesondere auch 2, 3, 4, 5, 6, 7, 8, und 9 mit umfasst. Liegt ein Wert zwischen 1,0 und 2,0 sind damit insbesondere auch 1,1, 1,2, 1,3, 1,4, 1,5, 1,6, 1,7, 1,8 und 1,9 mit umfasst. Liegt ein Wert zwischen 1,00 und 1,10 sind damit insbesondere auch 1,01, 1,02, 1,03, 1,04, 1,05, 1,06, 1,07, 1,08 und 1,09 mit umfasst.

### II. Verfahren

Die vorliegende Erfindung betrifft zunächst ein Verfahren zur Beschichtung von Oberflächen von Gegenständen, beispielswese Implantaten, mit einer Polymerschicht, in welcher mindestens ein pharmazeutischer Wirkstoff dispergiert ist. Das erfindungsgemäße Verfahren kann beispielsweise von medizinischem Fachpersonal unter geringem Aufwand ausgeführt werden, wobei ein Gegenstand erhalten wird, welcher mit einer Polymerschicht versehen ist, in der ein pharmazeutischer Wirkstoff dispergiert ist. Der Gegenstand kann dann an geeigneter Stelle, zum Beispiel *in-vivo*, über einen bestimmten Zeitraum den dispergierten pharmazeutischen Wirkstoff abgeben. Das erfindungsgemäße Verfahren umfasst die nachfolgenden Verfahrensschritte:
a) Auflösen eines nicht hydrolysierbaren Polymers oder Copolymers, welches durch eine zahlenmittlere Molmasse von größer als 200000 g/mol, weiter bevorzugt eine zahlenmittlere Molmasse von größer als 400000 g/mol, weiter bevorzugt eine zahlenmittlere Molmasse von größer als 600000 g/mol, bestimmt mittels Gelpermeations-Chromatografie gekennzeichnet ist, in einem Lösungsmittel oder Lösungsmittelgemisch, welches durch einen Dampfdruck von mindestens 95 hPa bei 20 °C und Normaldruck gekennzeichnet ist, unter Bildung einer Polymerlösung 1;
b) Auflösen oder Suspendieren von mindestens einem pharmazeutischen Wirkstoff in der Polymerlösung 1 unter Bildung einer Polymerlösung 2;
c) Auftragen der Polymerlösung 2 auf die Oberfläche eines zu beschichtenden Gegenstandes;
d) Verdunsten des Lösungsmittels oder Lösungsmittelgemischs, sodass auf dem zu beschichtenden Gegenstand eine Polymerschicht zurückbleibt, in welcher der mindestens eine pharmazeutische Wirkstoff dispergiert ist.
dadurch gekennzeichnet, dass die Polymerschicht keine Porenbildner aufweist.

Im Sinne der vorliegenden Erfindung wird ein nicht hydrolysierbares Polymer oder Copolymer ausgewählt, welches durch eine zahlenmittlere Molmasse von größer als 200000 g/mol, weiter bevorzugt eine zahlenmittlere Molmasse von größer als 400000 g/mol, weiter bevorzugt eine zahlenmittlere Molmasse von größer als 600000 g/mol, bestimmt mittels Gelpermeations-Chromatografie gekennzeichnet ist, und bevorzugt dazu geeignet ist, mit einem lebenden Organismus in Berührung zu kommen, ohne dass dem Organismus eine Schadwirkung zuteilwird.

Weiterhin ist erfindungsgemäß vorgesehen, dass das nicht hydrolysierbare Polymer oder Copolymer in einem Lösungsmittel oder Lösungsmittelgemisch, welches sich durch einen Dampfdruck von mindestens 95 hPa bei 20 °C und Normaldruck auszeichnet, gelöst oder suspendiert wird. Das Lösungsmittel oder Lösungsmittelgemisch im Sinne der vorliegenden Erfindung besitzt bevorzugt die Eigenschaft, bei üblichen Raumbedingungen wie 20 °C und Normaldruck verhältnismäßig schnell zu verdampfen. Dies ist von Vorteil, damit ein in dem Lösungsmittel oder Lösungsmittelgemisch gelöstes Polymer ohne zusätzlichen Aufwand, wie etwa einer Trocknung in einem Ofen, durch Auftragen der Polymerlösung verhältnismäßig schnell als Polymerbeschichtung auf der Oberfläche des Gegenstands verbleibt. Eine weitere bevorzugte Eigenschaft des Lösungsmittels oder Lösungsmittelgemisches im Sinne der vorliegenden Erfindung ist es, dass es mit einem lebenden Organismus in Berührung kommen kann, ohne dass dem Organismus eine Schadwirkung, wie eine toxische Wirkung zuteilwird.

Es ist also erfindungsgemäß insbesondere vorgesehen, wenn zunächst eine Polymerlösung 1 aus einem Polymer oder Copolymer mit einer zahlenmittleren Molmasse von größer als 200000 g/mol, weiter bevorzugt eine zahlenmittlere Molmasse von größer als 400000 g/mol, weiter bevorzugt eine zahlenmittlere Molmasse von größer als 600000 g/mol, bestimmt mittels Gelpermeations-Chromatografie, in einem geeigneten Lösungsmittel oder Lösungsmittelgemisch, welches durch einen Dampfdruck von mindestens 95 hPa bei 20 °C und Normaldruck gekennzeichnet ist, gebildet wird und keiner der genannten Stoffe der Polymerlösung 1 eine Schadwirkung, wie eine toxische Wirkung, auf lebende Organismen aufweist.

Geeignete nicht hydrolysierbare Polymere oder Copolymere für die Verwendung in dem erfindungsgemäßen Verfahren weisen bestimmte bevorzugte zahlenmittlere Molmassen (Mₙ) auf. So weisen bevorzugte Polymere oder Copolymere im Sinne der vorliegenden Erfindung eine zahlenmittlere Molmasse (Mₙ) von größer als 200000 g/mol auf, also beispielsweise eine zahlenmittlere Molmasse von größer als 200000 g/mol, 210000 g/mol, 220000 g/mol, 230000 g/mol, 240000 g/mol, 250000 g/mol, 260000 g/mol, 270000 g/mol, 280000 g/mol, 290000 g/mol, oder größer als 300000 g/mol, 310000 g/mol, 320000 g/mol, 330000 g/mol, 340000 g/mol, 350000 g/mol, 360000 g/mol, 370000 g/mol, 380000 g/mol, 390000 g/mol, oder größer als 400000 g/mol, 410000 g/mol, 420000 g/mol, 430000 g/mol, 440000 g/mol, 450000 g/mol, 460000 g/mol, 470000 g/mol, 480000 g/mol, 490000 g/mol, oder größer als 500000 g/mol, 510000 g/mol, 520000 g/mol, 530000 g/mol, 540000 g/mol, 550000 g/mol, 560000 g/mol, 570000 g/mol, 580000 g/mol, 590000 g/mol, oder größer als 600000 g/mol, auf.

Besonders bevorzugt ist die zahlenmittlere Molmasse der erfindungsgemäß zu verwendenden nicht hydrolysierbaren Polymere und Copolymere größer als 550000 g/mol.

Im Sinne der Erfindung wird die zahlenmittlere Molmasse (Mₙ) der Polymere oder Copolymere mittels der Gelpermeations-Chromatografie (GPC) bestimmt.

Erfindungsgemäß wurde festgestellt, dass nicht hydrolysierbare Polymere und Copolymere, die zahlenmittlere Molmassen von größer als 200000 g/mol aufweisen, zu Polymerschichten führen, die nicht zu klebrig sind und sich gut auf einer Oberfläche aufbringen lassen. Erfindungsgemäß wurde weiterhin festgestellt, dass Polymere und Copolymere, die durch die erfindungsgemäß bevorzugten zahlenmittleren Molmassen gekennzeichnet sind, hervorragend geeignet sind, um damit Polymerschichten zu produzieren, die an Oberflächen haften, nicht spröde oder rissig sind und pharmazeutische Wirkstoffe aufnehmen und abgeben können.

Erfindungsgemäß bevorzugt ist, dass nicht hydrolysierbare Polymere oder Copolymere für die Polymerlösung 1 verwendet werden, die eine Glasübergangstemperatur (Tg) von größer als 47 °C aufweisen. Bevorzugt weist das Polymer oder Copolymer für die Polymerlösung 1 eine Glasübergangstemperatur von größer als 60 °C, weiter bevorzugt größer als 80 °C, noch weiter bevorzugt größer als 100 °C, auf. Die bevorzugte Glasübergangstemperatur (Tg) für Polymere oder Copolymere, die erfindungsgemäß in der Polymerlösung 1 Anwendung finden, liegt also bei größer als 47 °C, 48 °C, 49 °C, oder bei größer als 50 °C, 51 °C, 52 °C, 53 °C, 54 °C, 55 °C, 56 °C, 57 °C, 58 °C, 59 °C, oder bei größer als 60 °C, 61 °C, 62 °C, 63 °C, 64 °C, 65 °C, 66 °C, 67 °C, 68 °C, 69 °C, oder bei größer als 70 °C, 71 °C, 72 °C, 73 °C, 74 °C, 75 °C, 76 °C, 77 °C, 78 °C, 79 °C, oder bei größer als 80 °C, 81 °C, 82 °C, 83 °C, 84 °C, 85 °C, 86 °C, 87 °C, 88 °C, 89 °C, oder bei größer als 90 °C, 91 °C, 92 °C, 93 °C, 94 °C, 95 °C, 96 °C, 97 °C, 98 °C, 99 °C oder bei größer als 100 °C.

Erfindungsgemäß konnte überraschend feststellt werden, dass nicht hydrolysierbare Polymere oder Copolymere mit Glasübergangstemperaturen (Tg) von größer als 47 °C, bevorzugt größer als 60 °C, bevorzugter größer als 80 °C, insbesondere größer als 100 °C, im Sinne der vorliegenden Erfindung geeignet sind, da diese Polymere oder Copolymere für einen Zeitraum von mindestens 7 Tage bei einer Temperatur von 37 °C in wässrigem Medium stabil blieben, ohne sich zu lösen. Wenn ein mittels des erfindungsgemäßen Verfahrens beschichteter Gegenstand *in-vivo* an einer geeigneten Wirkstelle mit dem in der Polymerschicht dispergierten mindestens einen pharmazeutischen Wirkstoff platziert wird, ist eine solche Wasser- und Temperaturbeständigkeit wünschenswert.

Die Glasübergangstemperatur (Tg) im Sinne der Erfindung wird über die Dynamische Differenzkalorimetrie (DSC) mit einer Heizrate von 10 K/min nach ISO 11357 beziehungsweise DIN 53765 bestimmt.

Es ist weiterhin erfindungsgemäß bevorzugt, dass nicht hydrolysierbare Polymere oder Copolymere, welche Bestandteil der erfindungsgemäßen Polymerlösung 1 sind, keine reaktiven Doppelbindungen enthalten. Reaktive Doppelbindungen bezeichnet solche Stellen in einem Polymergerüst, welche eine C-C Doppelbindung aufweisen und welche durch häufig auftretende äußere Einflüsse zu einer unerwünschten und unkontrollierten Reaktion gebracht werden können. Solche Doppelbindungen können beispielsweise bei der Einwirkung von Gammastrahlung in Gegenwart von Sauerstoff leicht oxidieren und es ist möglich, dass die Doppelbindungen zu Vernetzungen zwischen den Polymerketten führen. Diese Verletzungen können ein Gelieren der Polymerlösungen verursachen, sodass die Polymerlösungen nicht mehr fließfähig sind und daher nicht mehr gleichmäßig aufgetragen werden können. Im Umkehrschluss können die erfindungsgemäßen Polymerlösungen problemlos durch Gammabestrahlung sterilisiert werden, wenn keine Doppelbindungen in den gelösten Polymeren enthalten sind, was für medizinische Anwendungsbereiche einen erfindungsgemäßen Vorteil darstellt.

Eine erfindungsgemäß bevorzugte weitere Eigenschaft der nicht hydrolysierbare Polymere oder Copolymere, die im erfindungsgemäßen Verfahren Bestandteil der Polymerlösung 1 sind, ist, dass die aus ihnen hergestellte Polymerschicht nicht zu steif und zu spröde ist, sie also beweglich bleibt und auch bei gegebenenfalls auftretenden Verformungen des beschichteten Gegenstandes nicht abbröckelt.

Im Sinne der vorliegenden Erfindung wurde gefunden, dass insbesondere hydrophobe Polymere und Copolymere mit den erfindungsgemäßen Eigenschaften zur Ausführung des erfindungsgemäßen Verfahrens geeignet sind. Erfindungsgemäß wurde überraschend herausgefunden, dass nicht hydrolysierbare Polymere und Copolymere von Polymethylmethacrylaten sich besonders zur Bildung der Polymerschichten im Rahmen der vorliegenden Erfindung eignen. Insbesondere solche nicht hydrolysierbaren Polymere und Copolymere von Polymethylmethacrylaten, die durch die erfindungsgemäß bevorzugten Bereiche von Glasübergangstemperaturen und gegebenenfalls zahlenmittleren Molmassen gekennzeichnet sind, eignen sich zur Umsetzung des erfindungsgemäßen Verfahrens. Weiterhin bevorzugt werden insbesondere solche nicht hydrolysierbaren Polymere und Copolymere von Polymethylmethacrylaten eingesetzt, die keine kristallinen Bestandteile aufweisen, also bevorzugt vollständig amorph sind. Die starken intermolekularen Wechselwirkungen, die üblicherweise in kristallinen Verbindungen eine Rolle spielen, würden die Eigenschaften der Polymerschicht negativ beeinflussen, indem sie zu erhöhter Sprödigkeit und Rissbildung führen könnten. Die bevorzugt amorphen, weiter bevorzugt nicht hydrolysierbaren Polymere und Copolymere im Sinne der Erfindung bilden hingegen schwächere, dispersive Wechselwirkungen, wie beispielsweise Dipol-Wechselwirkungen und Van-der-Waals Wechselwirkungen aus, die makroskopisch zu einer formbareren Substanz ohne Sprödigkeit und Risse führen. Dadurch sind die Polymere und Copolymere mit den erfindungsgemäßen Eigenschaften für das erfindungsgemäße Verfahren zur Bildung einer Polymerschicht besonders geeignet.

Nicht hydrolysierbare Polymere und Copolymere, die von Polymethylmethacrylaten abgeleitet sind und sich durch die erfindungsgemäß bevorzugten Eigenschaften kennzeichnen, sind im Sinne des erfindungsgemäßen Verfahrens bevorzugt, da sie über eine hervorragende Biokompatibilität verfügen. Sie können also *in-vivo* eingesetzt werden, was bevorzugt ist, da die Polymerschichten, die mit dem erfindungsgemäßen Verfahren gebildet werden, bevorzugt für *in-vivo* Anwendung verwendet werden. Die Polymere und Copolymere, die von Polymethylmethacrylaten abgeleitet sind, sind nicht cytotoxisch und hydrolysefest.

Wenn im Sinne der vorliegenden Erfindung von Copolymeren des Polymethylmethacrylats gesprochen wird, werden darunter insbesondere Copolymere verstanden, die aus Methylmethacrylat und den Comonomeren Methylacrylat, Ethylacrylat, Ethylmethacrylat und Styren durch Polymerisation hergestellt werden können.

Im Sinne der vorliegenden Erfindung wurde festgestellt, dass durch den Einsatz von nicht hydrolysierbaren Polymeren und Copolymeren mit den erfindungsgemäß bevorzugten Eigenschaften Polymerbeschichtungen erhalten werden, welche die gewünschte Sprödigkeit aufweisen, einen hohen Gehalt an pharmazeutischen Wirkstoffen aufnehmen können und den pharmazeutischen Wirkstoff am Zielort zuverlässig abgeben.

Eine erfindungsgemäße Besonderheit hierbei ist, dass die nicht hydrolysierbaren Polymere und Copolymere dabei ohne Zusätze auskommen, die im Stand der Technik üblicherweise zwingend hinzugesetzt werden müssen, wie insbesondere Porenbildner.

Porenbildner im Sinne der vorliegenden Erfindung sind insbesondere Bestandteile in der Polymerschicht, welche Poren in der wasserunlöslichen Polymerschicht unter Einwirkung von Wasser erzeugen können. Porenbildner sind ferner insbesondere Stoffe, welche wasserlöslich und/oder hydrolysierbar sind. Beispielhafte Porenbildner umfassen Poly-α-hydroxysäuren oder Oligomere zur Herstellung von Poly-α-hydroxysäuren, insbesondere Polyglykole, Polylactide, wie D,L-Polylactide, Polytyrosincarbonate und oligomere Ethylenglykolverbindungen, insbesondere oligomere Ethylenglykolverbindungen mit einer zahlenmittleren Molmasse im Bereich von 120 g/mol bis 35000 g/mol, Stärke, Gelatine, Cellulose, α-Hydroxysäuren, wie Milchsäure, besonders D,L-Milchsäure, Zuckeralkohole, wie Ethylenglykol und Diethylenglykol, Mannitol, Xylol und Sorbitol, Aminosäuren, wie Alanin, Glycin, Tyrosin und Serin, und Tenside, wie Fettalkohole.

Porenbildner umfassen insbesondere Stoffe, welche in einer Konzentration von 1 g/Liter in Phosphat-gepufferter Salzlösung (d.h. 137 mM Natriumchlorid, 2,7 mM Kaliumchlorid und 12 mM Gesamt-Phosphat, pH 7,4) bei 25 °C innerhalb von 24 Stunden im Wesentlichen vollständig löslich sind und/oder zersetzt werden.

Im Stand der Technik bekannte Verfahren zur Herstellung einer Polymerbeschichtung mit einem darin dispergierten pharmazeutischen Wirkstoff erfordern, dass der Polymerbeschichtung weitere Zusätze wie Porenbildner beigefügt werden. Dadurch wird die Polymerbeschichtung so modifiziert, dass sie pharmazeutische Wirkstoffe in ausreichender Menge aufnehmen und am Zielort wieder abgeben kann. Das erfindungsgemäße Verfahren führt zu einer Polymerbeschichtung, die solche Zusätze nicht erfordert.

Insgesamt sind insbesondere nicht-kristalline, hydrophobe Polymere oder Copolymere von Polymethylmethacrylaten mit zahlenmittleren Molmassen (Mₙ) von größer als 200000 g/mol, weiter bevorzugt größer als 400000 g/mol, weiter bevorzugt größer als 600000 g/mol, und Glasübergangstemperaturen (Tg) von größer als 47 °C, weiter bevorzugt größer als 60 °C, weiter bevorzugt größer als 80 °C, weiter bevorzugt größer als 100 °C, im Sinne der vorliegenden Erfindung besonders bevorzugt.

Weiterhin ist es erfindungsgemäß bevorzugt, ein Lösungsmittel oder Lösungsmittelgemisch zur Herstellung der erfindungsgemäßen Polymerlösung 1 zu verwenden, welches weniger als 8 Gew.-% Wasser aufweist. Das Lösungsmittel oder Lösungsmittelgemisch weist weiter bevorzugt weniger als 7 Gew.-%, weniger als 6 Gew.-%, weniger als 5 Gew.-%, weniger als 4 Gew.-%, weniger als 3 Gew.-%, weniger als 2 Gew.-%, Wasser auf. Eine Verunreinigung des Lösungsmittels oder Lösungsmittelgemisches mit Wasser kann bei hygroskopischen pharmazeutisch Wirkstoffen zu einer Verklumpung der Wirkstoffpartikel in der Polymerlösung 2 führen. Daher ist ein möglichst niedriger Wassergehalt von weniger als 2 Gew.-% besonders bevorzugt.

Es ist erfindungsgemäß von Vorteil, wenn das Lösungsmittel oder Lösungsmittelgemisch an das Polymer oder Copolymer für die Polymerlösung 1 angepasst wird; das heißt, dass vorzugsweise ein Lösungsmittel oder Lösungsmittelgemisch verwendet wird, um die Löslichkeit bestimmter Monomere oder Comonomere für das Polymer oder Copolymer zu erhöhen.

Im Sinne des erfindungsgemäßen Verfahrens umfassen Lösungsmittel oder Lösungsmittelgemische bevorzugt Essigsäureester, Acetessigsäureester, Aceton, Diacetonalkohol, Ethylmethylketon, Butylmethylketon und/oder Mischungen davon. Auch bevorzugt im Sinne der Erfindung ist es, Mischungen mit einem Essigsäureester und einem anderen Lösungsmittel, umfassend Acetessigsäureester, bevorzugt Acetessigsäureethylester, Aceton, Diacetonalkohol, Ethylmethylketoon und/oder Butylmethylketon, zu verwenden. Ein besonders bevorzugtes Lösungsmittel im Sinne der vorliegenden Erfindung ist ein Essigsäureester, insbesondere Essigsäureethylester.

Essigsäureethylester ist nicht toxisch, hat einen Dampfdruck von 98,4 hPa bei 20 °C und Normaldruck und einen Siedepunkt von 77 °C bei Normaldruck. Essigsäureethylester verdunstet daher verhältnismäßig schnell bei Raumtemperatur. Eine weitere vorteilhafte Eigenschaft von Essigsäureethylester ist es, dass dessen Geruch von den meisten Menschen als angenehm wahrgenommen wird. Wenn also im Sinne des erfindungsgemäßen Verfahrens ein polymerbeschichteter Gegenstand hergestellt wird, der gegebenenfalls für eine medizinische *in-vivo* Anwendung geeignet sein muss, ist der Essigsäureethylester neben seinen ausgezeichneten physikalischen und toxikologischen Qualitäten auch hinsichtlich der olfaktorischen Wahrnehmung von Fachpersonal und gegebenenfalls Patient bevorzugt. Essigsäureethylester ist außerdem einfach zu handhaben und beschädigt im Gegensatz zu anderen Lösungsmitteln mit ähnlichen Eigenschaften die meisten typischen Plastikgegenständen, die in dem weiter unten beschriebenen erfindungsgemäßen Kit-of-parts verwendet werden, nicht.

Erfindungsgemäße wurde überraschend festgestellt, dass Lösungen von erfindungsgemäß vorgeschlagenen Polymeren und Copolymeren in Essigsäureethylester sehr gut haftende und stabile Polymerschichten ausbilden, wenn diese auf Oberflächen von Gegenständen, vorzugsweise metallischen Oberflächen von Gegenständen, aufgetragen werden und das Lösungsmittel verdunstet. Der Verdunstungsprozess läuft im Wesentlichen innerhalb von ein bis zwei Minuten bei einer Raumtemperatur von 23 °C ab. Damit eignet sich Essigsäureethylester im Sinne der Erfindung besonders gut, um die Beschichtungen ohne größeren Aufwand und verhältnismäßig schnell auszuführen.

Die Polymerlösung 1, umfassend ein erfindungsgemäß bevorzugtes Polymer oder Copolymer und ein erfindungsgemäß vorgeschlagenes Lösungsmittel oder Lösungsmittelgemisch, weist bevorzugt einen Polymergehalt zwischen 2 Gew.-% und 18 Gew.-%, also beispielsweise mindestens 2 Gew.%, mindestens 3 Gew.%, mindestens 4 Gew.%, mindestens 5 Gew.%, mindestens 6 Gew.%, mindestens 7 Gew.%, mindestens 8 Gew.%, mindestens 9 Gew.%, bevorzugt mindestens 10 Gew.%, auf. Erfindungsgemäß ist auch ein Polymergehalt von weniger gleich 18 Gew.%, weniger gleich 17 Gew.%, weniger gleich 16 Gew.%, weniger gleich 15 Gew.%, weniger gleich 14 Gew.%, weniger gleich 13 Gew.%, weniger gleich 12 Gew.%, weniger gleich 11 Gew.%, bevorzugt.

Besonders bevorzugt ist ein Polymergehalt von 9 Gew.-% bis 11 Gew.%. Bei dieser Polymerkonzentration lässt sich die Polymerlösung leicht mit pharmazeutischen Wirkstoffen vermischen und anschließend einfach auf Oberflächen auftragen.

Höhere Polymerkonzentration als 18 Gew.-% sind möglich, aber mit zunehmender Polymerkonzentration können sich Eigenschaften, wie die Streich- oder Sprühfähigkeit der Polymerlösung, verschlechtern, da die Polymerlösung dadurch gegebenenfalls klumpig oder zäh wird.

Ist die Polymerkonzentration hingegen zu gering, kann sich dies negativ auf die Polymerschichtbildungseigenschaften der Polymerlösung 2 auswirken. Das bedeutet beispielsweise, dass sich keine abschließende Polymerschicht beim Auftragen einer Polymerlösung 2 mit zu geringer Polymerkonzentration bildet, sondern eine Polymerschicht, die Löcher aufweist.

Im Sinne der vorliegenden Erfindung wird mindestens ein pharmazeutischer Wirkstoff in der erhaltenen Polymerlösung 1 aufgelöst oder dispergiert. Erfindungsgemäß ist dabei prinzipiell jeder pharmazeutische Wirkstoff geeignet, der mit einer Polymerlösung 1 in Berührung gebracht werden kann, ohne sich zu zersetzen. Es ist erfindungsgemäß möglich, dass sich der pharmazeutische Wirkstoff in der Polymerlösung 1 auflöst oder in der Polymerlösung 1 suspendiert wird.

Bevorzugt wird der pharmazeutische Wirkstoff in der Polymerlösung 1 suspendiert. Viele aktuell verfügbare pharmazeutische Wirkstoffe weisen nur eine geringe Löslichkeit in üblichen Lösungsmitteln auf. Daraus resultiert häufig eine geringe Konzentration des pharmazeutischen Wirkstoffs in einer Lösung des Polymers, wodurch wiederum eine geringe Konzentration des pharmazeutischen Wirkstoffs in einer Beschichtung, erhaltend ausgehend von einer Lösung, resultiert. Damit ist die Bioverfügbarkeit in der Anwendung gering und es stellt ein Problem dar, den pharmazeutischen Wirkstoff in ausreichender Menge zum gewünschten Wirkort zu transportieren. Daher sind Suspensionen des pharmazeutischen Wirkstoffs in dem Lösungsmittel oder Lösungsmittelgemisch bevorzugt, da sich hierdurch höhere Konzentrationen realisieren lassen.

Im Sinne der vorliegenden Erfindung wird die Lösung oder Suspension, die erhalten wird, wenn ein pharmazeutischer Wirkstoff in einer erfindungsgemäßen Polymerlösung 1 gelöst oder suspendiert wird, als Polymerlösung 2 bezeichnet, auch dann, wenn es sich dabei um eine Suspension handelt.

Im Sinne der vorliegenden Erfindung wird anschließend die Polymerlösung 2, umfassend das Lösungsmittel oder Lösungsmittelgemisch, das gelöste Polymer oder Copolymer und der mindestens eine gelöste oder suspendierte pharmazeutische Wirkstoff, auf der Oberfläche eines Gegenstandes aufgetragen, auf welcher eine Polymerschicht mit dem darin dispergierten pharmazeutischen Wirkstoff erhalten werden soll.

Nach dem Auftragen verdunstet das ausgewählte Lösungsmittel bevorzugt verhältnismäßig schnell bei raumüblichen Bedingungen, und zwar bevorzugt, ohne dass ein zusätzlicher Aufwand. wie ein Trocknen in einem geeigneten Ofen oder eine Trocknung mit einem Heißluftfön, erforderlich ist. Zurück bleibt ein Gegenstand mit einer Polymerschicht, in welcher der pharmazeutische Wirkstoff dispergiert ist.

Im Sinne des erfindungsgemäßen Verfahrens wird in der Polymerlösung 1 mindestens ein pharmazeutischer Wirkstoff gelöst oder suspendiert, um eine Polymerlösung 2 zu erhalten. In Frage kommende pharmazeutische Wirkstoffe umfassen beispielsweise Wirkstoffe aus den Klassen der Antiinfektiva, Bakteriophagen, Cytostatika, Analgetika, Hormone und Wachstumsfaktoren. Gegenstände, die mit der Polymerlösung 2 beschichtet werden, können dann Wirkstoffe, insbesondere aus den aufgezählten Wirkstoffklassen, lokal an einem Wirkort, auch *in-vivo*, über einen bestimmten Zeitraum kontinuierlich freisetzen.

Unter dem Begriff "Antiinfektiva" werden antimikrobiell aktive Wirkstoffe verstanden, die mikrobiellen, halbsynthetischen und auch synthetischen Ursprungs sein können.

Im Sinne der Erfindung bezeichnet der Begriff "Bakteriophagen" Wirkstoffe, die gezielt zu therapeutischen Zwecken antimikrobiell eingesetzt werden.

Der Begriff Cytostatika bezeichnet solche Wirkstoffe, die eine toxische Wirkung auf Zellen aufweisen und üblicherweise zur Behandlung von Krebserkrankungen eingesetzt werden.

Mit dem Begriff Analgetika sind solche Wirkstoffe gemeint, die eine schmerzstillende oder schmerzlindernde Wirkung besitzen.

Hormone und Wachstumsfaktoren sind körpereigene Substanzen oder von körpereigenen Substanzen abgeleitete Wirkstoffe, die körpereigene Prozesse, etwa die des Stoffwechsels, beeinflussen können.

Im Sinne des erfindungsgemäßen Verfahrens liegen besonders bevorzugt pharmazeutische Wirkstoffe wie Gentamicin, Tobramycin, Amikacin, Clindamycin, Colistin, Moxifloxacin, Levofloxacin, Ciprofloxacin, Vancomycin, Teicoplanin, Daptomycin, Doxycyclin, Ampicillin, Amoxicillin, Meropenem, Fluconazol, Amphotericin B, Caspofungin, Micafungin und Mischungen der vorgenannten Wirkstoffe in der Polymerlösung 2 gelöst oder suspendiert vor.

Es ist im Sinne des vorliegenden erfindungsgemäßen Verfahrens ganz besonders bevorzugt, wenn die pharmazeutischen Wirkstoffe vor dem Auflösen oder Suspendieren in einer partikulären Form vorliegen; das heißt, dass die Wirkstoffe als Feststoff vorliegen, der durch bestimmte Partikelgrößenbereiche charakterisiert ist. Bevorzugterweise liegen die pharmazeutischen Wirkstoffe vor dem Auflösen oder Suspendieren mit einer Partikelgröße von kleiner als 250 µm, bevorzugt von kleiner als 100 µm, vor. Die Partikelgröße beträgt insbesondere weniger als 240 µm, weniger als 230 µm, weniger als 220 µm, weniger als 210 µm, weniger als 200 µm, weniger als 190 µm, weniger als 180 µm, weniger als 170 µm, weniger als 160 µm, weniger als 150 µm, weniger als 140 µm, weniger als 130 µm, weniger als 120 µm, weniger als 110 µm, vor. Im Sinne der Erfindung wird die Partikelgröße durch einen Siebvorgang (Siebfraktionierung) bestimmt.

Erfindungsgemäß wurde festgestellt, dass besonders fein gesiebte partikuläre pharmazeutische Wirkstoffe zu bevorzugen sind, da sie in Form einer Suspension die Bildung einer gleichmäßigen Polymerbeschichtung fördern. Größere Partikel neigen zur Verklumpung oder zur Bildung einer ungleichmäßigen Beschichtung, was erfindungsgemäß nicht bevorzugt ist. Wird ein Auflösen der pharmazeutischen Wirkstoffe angestrebt, sind kleine Partikel ebenfalls bevorzugt, denn kleinere Partikel lösen sich prinzipiell schneller auf als Größere, da sie ein ungünstiges Verhältnis von Oberfläche zu Volumen aufweisen.

Um die Bildung einer gleichmäßigen Suspension oder einer vollständigen Auflösung des pharmazeutischen Wirkstoffs bei der Ausführung des erfindungsgemäßen Verfahrens zu fördern, ist es bevorzugt, den Auflöse- oder Suspensionsvorgang mit einem Mischkörper zu unterstützen. Dabei können beispielsweise Mischstäbe, zum Beispiel laborübliche Glas- oder Rührstäbe, verwendet werden, um den pharmazeutischen Wirkstoff in der Polymerlösung 1 bis zum Auflösen oder bis zur gleichmäßigen Suspension zur Polymerlösung 2 zu verrühren. Es ist ebenfalls denkbar, Mischelemente, wie Kugeln aus Glas, Keramik, Metall oder einem anderen Material oder als Rührfischchen oder Rührstäbchen bezeichnete magnetische Rührelemente, zu verwenden. Im Falle der diversen Kugeln kann bevorzugt ein geschlossenes Behältnis, umfassend die Kugeln, Polymerlösung 1 und den pharmazeutischen Wirkstoff, verwendet werden, welches dann von Hand oder mit einer Schüttelvorrichtung geschüttelt wird. Mithilfe des magnetischen Rührelements kann die Mischung aus Polymerlösung 1 und pharmazeutischem Wirkstoff auf einem Magnetrührer gerührt werden.

Die Konzentration an pharmazeutischem Wirkstoff, die in einer mittels des erfindungsgemäßen Verfahrens erhältlichen Polymerbeschichtung erreicht werden soll, hängt physikalisch im Wesentlichen vom Polymergehalt in der Polymerlösung 2 ab. Dabei herrscht in der Polymerlösung 2 bevorzugt dasselbe Verhältnis zwischen Polymer oder Copolymer und pharmazeutischem Wirkstoff vor, das auch in der finalen Polymerbeschichtung erhalten werden soll. In dem erfindungsgemäßen Verfahren wird ein Gewichtsverhältnis zwischen Polymeren oder Copolymeren und pharmazeutischen Wirkstoffen von vorzugsweise zwischen 1,00 zu 0,05 und 1,00 zu 1,50 verwendet. Das bedeutet, dass das Gewichtsverhältnis zwischen Polymer(en) und Copolymer(en) und pharmazeutischem Wirkstoff(en) beispielsweise bei 1,00 zu 0,05 bis 1,00 zu 1,50, bei 1,00 zu 0,30 bis 1,00 zu 1,30, bei 1,00 zu 0,60 bis 1,00 zu 1,15 oder bei 1,00 zu 0,95 bis 1,00 zu 1,05, liegt. Überraschend ist, dass es mit dem erfindungsgemäßen Verfahren gelingt, Beschichtungen herzustellen, deren Wirkstoffgehalt größer ist als deren Polymergehalt. Dadurch können hohe Beladungsdichten auf der beschichteten Oberfläche erzielt werden.

Ein erfindungsgemäßer Vorteil ist es, dass die erfindungsgemäßen Polymerbeschichtungen eine sehr hohe Beladungsdichten mit pharmazeutisch aktivem Wirkstoff aufweisen können, wobei es dennoch nicht erforderlich ist, den Polymerbeschichtungen Zusätze wie Porenbildner hinzuzufügen, damit die Schicht am Zielort stabil bleibt und den pharmazeutischen Wirkstoff kontinuierlich abgibt. Es kann also eine überraschend hohe Beladungsdichte mit einem pharmazeutischen Wirkstoff in der Polymerschicht erreicht werden, ohne dass die im Stand der Technik üblichen Bestandteile verwendet werden müssen. Es können also durch das erfindungsgemäße Verfahren Polymerschichten erhalten werden, die hervorragende Haftungseigenschaften aufweisen, nicht spröde sind und hohe Mengen an pharmazeutischen Wirkstoffen enthalten, ohne dass sich dies negativ auf die Beschaffenheit der Polymerbeschichtung auswirkt. Daher sind erfindungsgemäß Verhältnisse zwischen Polymer(en) und Copolymer(en) und pharmazeutischem Wirkstoff(en) bei 1,00 zu größer gleich 1,00 bevorzugt.

Wenn eine erfindungsgemäße Polymerlösung 2 entsprechend den zuvor beschriebenen Schritten hergestellt wurde, kann sie im Sinne des erfindungsgemäßen Verfahrens prinzipiell auf alle Arten von Gegenständen aufgetragen werden, um eine Polymerschicht darauf zu erzeugen, in welcher ein pharmazeutischer Wirkstoff dispergiert ist. Im Sinne des erfindungsgemäßen Verfahrens ist es besonders bevorzugt, wenn das Auftragen mit einer einfachen Methode, die keinen größeren Aufwand bedarf, ausgeführt wird, beispielsweise durch Streichen der Polymerlösung 2 auf eine Oberfläche, durch Eintauchen eines zu beschichtenden Gegenstands in ein Behältnis mit der Polymerlösung 2 oder durch Aufsprühen der Polymerlösung 2 auf eine Oberfläche.

Im Sinne des vorliegenden Verfahrens ist es weiterhin bevorzugt, wenn die Oberfläche des zu beschichtenden Gegenstandes bei der Auftragung der Polymerlösung 2 eine Temperatur im Bereich von 10°C bis 30 °C aufweist, also beispielsweise eine Temperatur von 10 °C bis 30 °C, von 10 °C bis 28 °C, von 10 °C bis 26 °C, von 10 °C bis 24 °C, von 10 °C bis 22 °C, von 10 °C bis 20 °C, aufweist.

Bevorzugt liegt die Temperatur der Oberfläche des zu beschichtenden Genstandes bei 10 °C bis 30 °C. Bei tieferen Temperaturen als 10 °C benötigt das Lösungsmittel oder Lösungsmittelgemisch gegebenenfalls mehrere Minuten zum Verdunsten, was im Sinne der vorliegenden Erfindung nicht bevorzugt ist. Bei Temperaturen größer 30 °C wurde erfindungsgemäß herausgefunden, dass das Lösungsmittel oder Lösungsmittelgemisch gegebenenfalls zu schnell verdunstet und es schwierig ist, durch Auftragen der Polymerlösung 2 gleichmäßige Schichten auf die Oberfläche aufzubringen.

Mit dem erfindungsgemäßen Verfahren lassen sich gleichmäßige Beschichtungen herstellen. Gleichmäßige Beschichtungen im Sinne der vorliegenden Erfindung bedeuten eine vollständige Bedeckung der Oberfläche des Gegenstandes mit der aus der Polymerlösung 2 resultierenden Beschichtung. Das bedeutet weiterhin, dass die Polymerbeschichtungen vorzugsweise keine Risse oder optische Anzeichen von Sprödigkeit aufweisen und fest genug auf der Gegenstandsoberfläche haften, um nicht ohne Kraftaufwand oder Einwirkung von chemischen oder physikalischen Mitteln wieder abgelöst werden zu können.

Die erfindungsgemäß erhaltenen Polymerbeschichtungen, in denen pharmazeutische Wirkstoffe dispergiert sind, weisen eine Schichtdicke von größer als 50 µm auf. Insbesondere sind die Schichtdicken größer als 60 µm, größer als 70 µm, größer als 80 µm, größer als 90 µm, größer als 100 µm, größer als 110 µm, größer als 120 µm, größer als 130 µm, größer als 140 µm oder größer als 150 µm. Die Schichtdicken können aber auch bis zu 500 µm, bis zu 450 µm, bis zu 400 µm, bis zu 350 µm oder bis zu 300 µm dick sein. Die Beschichtungen, die mittels des erfindungsgemäßen Verfahrens auf einer Oberfläche erhalten werden, weisen insbesondere keine Risse oder Anzeichen von Sprödigkeit auf und sind gleichmäßig dick.

Erfindungsgemäß wurde festgestellt, dass solche verhältnismäßig dünnen Beschichtungen, wie sie mit dem erfindungsgemäßen Verfahren erhalten werden können, die Freisetzung des in den Beschichtungen dispergierten pharmazeutischen Wirkstoffes am Wirkungsort erleichtern. Im Gegensatz zu im Stand der Technik üblichen Beschichtungen ist dabei kein Zusatz einer hydrophilen Komponente, wie einem Polyether, in das Polymer oder Copolymer erforderlich.

Im Sinne des erfindungsgemäßen Verfahrens können prinzipiell alle Gegenstände, insbesondere Gegenstände zur Verwendung in medizinischen Anwendungen, weiter insbesondere medizinische Implantate, mit einer Polymerbeschichtung, die einen oder mehrere pharmazeutische Wirkstoffe enthält, beschichtet werden.

Mit dem erfindungsgemäßen Verfahren ist es also beispielsweise möglich, ein medizinisches Implantat, zum Beispiel einen intramedullären Nagel oder eine Osteosyntheseplatte, mit einer pharmazeutisch aktiven Polymerbeschichtung zu beschichten. Das Verfahren ist aufgrund der leicht handhabbaren Bestandteile für die Polymerlösungen 1 und 2 ohne größeren Aufwand ausführbar und liefert innerhalb von Minuten beschichtete Implantate. Diese Implantate können eingesetzt werden und geben direkt am Zielort kontinuierlich Wirkstoff ab.

Im Folgenden kann das erfindungsgemäße Verfahren insbesondere im Rahmen der in Tabelle 1 definierten Parameter ausgeführt werden:
a) Auflösen eines nicht hydrolysierbaren Polymers oder Copolymers mit einer zahlenmittleren Molmasse (Mₙ) **A** und einer Glasübergangstemperatur (Tg) **B** in einem Lösungsmittel **C** oder Lösungsmittelgemisch aus mehr als einem Lösungsmittel **C** unter Bildung einer Polymerlösung 1;
b) Auflösen oder Suspendieren von mindestens einem pharmazeutischen Wirkstoff in der Polymerlösung 1, unter Bildung einer Polymerlösung 2;
c) Auftragen der Polymerlösung 2 auf die Oberfläche eines Gegenstandes; und
d) Verdunsten des Lösungsmittels oder Lösungsmittelgemischs, sodass auf der Gegenstandsoberfläche eine Polymerschicht zurückbleibt, in welcher der mindestens eine pharmazeutische Wirkstoff dispergiert ist.
dadurch gekennzeichnet, dass die Polymerschicht keine Porenbildner aufweist.

**Tabelle 1. Auswahl bevorzugter zahlenmittlerer Molmassen (Mₙ) A bevorzugter Glasübergangstemperaturbereiche (Tg) B, , bevorzugter Lösungsmittel C, die einzeln oder in Form von Lösungsmittelgemischen verwendet werden.**

| **Ausgestaltung** | **Mₙ [g/mol] A** | **Tg [°C] B** | **Lösungsmittel C** |
|---|---|---|---|
| 1 | > 200000 | > 47 | Essigsäureethylester |
| | | | Acetessigsäureethylester |
| | | | Aceton |
| | | | Diacetonalkohol |
| | | | Ethylmethylketon |
| | | | Butylmethylketon |
| 2 | > 200000 | > 60 | Essigsäureethylester |
| | | | Acetessigsäureethylester |
| | | | Aceton |
| | | | Diacetonalkohol |
| | | | Ethylmethylketon |
| | | | Butylmethylketon |
| 3 | > 200000 | > 80 | Essigsäureethylester |
| | | | Acetessigsäureethylester |
| | | | Aceton |
| | | | Diacetonalkohol |
| | | | Ethylmethylketon |
| | | | Butylmethylketon |
| 4 | > 200000 | > 100 | Essigsäureethylester |
| | | | Acetessigsäureethylester |
| | | | Aceton |
| | | | Diacetonalkohol |
| | | | Ethylmethylketon |
| | | | Butylmethylketon |
| 5 | > 400000 | > 47 | Essigsäureethylester |
| | | | Acetessigsäureethylester |
| | | | Aceton |
| | | | Diacetonalkohol |
| | | | Ethylmethylketon |
| | | | Butylmethylketon |
| 6 | > 400000 | > 60 | Essigsäureethylester |
| | | | Acetessigsäureethylester |
| | | | Aceton |
| | | | Diacetonalkohol |
| | | | Ethylmethylketon |
| | | | Butylmethylketon |
| 7 | > 400000 | > 80 | Essigsäureethylester |
| | | | Acetessigsäureethylester |
| | | | Aceton |
| | | | Diacetonalkohol |
| | | | Ethylmethylketon |
| | | | Butylmethylketon |
| 8 | > 400000 | > 100 | Essigsäureethylester |
| | | | Acetessigsäureethylester |
| | | | Aceton |
| | | | Diacetonalkohol |
| | | | Ethylmethylketon |
| | | | Butylmethylketon |
| 9 | > 600000 | > 47 | Essigsäureethylester |
| | | | Acetessigsäureethylester |
| | | | Aceton |
| | | | Diacetonalkohol |
| | | | Ethylmethylketon |
| | | | Butylmethylketon |
| 10 | > 600000 | > 60 | Essigsäureethylester |
| | | | Acetessigsäureethylester |
| | | | Aceton |
| | | | Diacetonalkohol |
| | | | Ethylmethylketon |
| | | | Butylmethylketon |
| 11 | > 600000 | > 80 | Essigsäureethylester |
| | | | Acetessigsäureethylester |
| | | | Aceton |
| | | | Diacetonalkohol |
| | | | Ethylmethylketon |
| | | | Butylmethylketon |
| 12 | > 600000 | > 100 | Essigsäureethylester |
| | | | Acetessigsäureethylester |
| | | | Aceton |
| | | | Diacetonalkohol |
| | | | Ethylmethylketon |
| | | | Butylmethylketon |

Nachfolgend wird eine besonders bevorzugte Ausführungsformen des erfindungsgemäßen Verfahrens beschrieben. In dieser besonders bevorzugten Ausführungsform ist das erfindungsgemäße Verfahren durch die nachfolgenden Verfahrensschritte gekennzeichnet:
a) Auflösen eines Polymers oder Copolymers, welches durch eine zahlenmittlere Molmasse von größer als 200000 g/mol gekennzeichnet ist, in einem Essigsäureester oder einem Gemisch eines Essigsäureesters mit mindestens einem weiteren Lösungsmittel, welche durch einen Dampfdruck von mindestens 95 hPa bei 20 °C und Normaldruck gekennzeichnet sind, unter Bildung einer Polymerlösung 1;
b) Auflösen oder Suspendieren von mindestens einem partikulären Antiinfektivum in der Polymerlösung 1 unter Bildung einer Polymerlösung 2;
c) Aufstreichen der Polymerlösung 2 auf die Oberfläche eines medizinischen Implantates; und
d) Verdunsten des Lösungsmittels oder Lösungsmittelgemischs, sodass auf der Implantatoberfläche eine Polymerschicht zurückbleibt, in welcher das mindestens eine partikuläre Antiinfektivum dispergiert ist.
dadurch gekennzeichnet, dass die Polymerschicht keine Porenbildner aufweist.

Eine weitere besonders bevorzugte Ausführungsform des erfindungsgemäßen Verfahrens umfasst insbesondere die nachfolgenden Verfahrensschritte:
a) Auflösen eines Polymethylmethacrylates oder eines Polymethyl-co-methylacrylates, welches durch eine zahlenmittlere Molmasse von größer als 200000 g/mol gekennzeichnet ist, in einem Essigsäureester oder einem Gemisch eines Essigsäureesters mit mindestens einem weiteren Lösungsmittel, welche durch einen Dampfdruck von mindestens 95 hPa bei 20 °C und Normaldruck gekennzeichnet sind, unter Bildung einer Polymerlösung 1;
b) Auflösen oder Suspendieren von mindestens einem partikulären Antiinfektivum in der Polymerlösung 1 unter Bildung einer Polymerlösung 2;
c) Aufstreichen der Polymerlösung 2 auf die Oberfläche eines medizinischen Implantates;
d) Verdunsten des Lösungsmittels oder Lösungsmittelgemischs, sodass auf der Implantatoberfläche eine Polymerschicht zurückbleibt, in welcher das mindestens eine partikuläre Antiinfektivum dispergiert ist.
dadurch gekennzeichnet, dass die Polymerschicht keine Porenbildner aufweist.

Eine weitere besonders bevorzugte Ausführungsform des erfindungsgemäßen Verfahrens umfasst insbesondere die nachfolgenden Verfahrensschritte:
a) Auflösen eines Polymethylmethacrylates oder eines Polymethyl-co-methylacrylates, welches durch eine zahlenmittlere Molmasse von größer als 200000 g/mol gekennzeichnet ist, in Essigsäureethylester oder einem Gemisch von Essigsäureethylester mit mindestens einem weiteren Lösungsmittel, welche durch einen Dampfdruck von mindestens 95 hPa bei 20 °C und Normaldruck gekennzeichnet sind, unter Bildung einer Polymerlösung 1;
b) Auflösen oder Suspendieren von mindestens einem partikulären Antiinfektivum ausgewählt aus der Gruppe, bestehend aus Gentamicinsulfat, Vancomycinhydrochlorid, Clindamycinhydrochlorid, Daptomycin und Mischungen davon, in der Polymerlösung 1 unter Bildung einer Polymerlösung 2;
c) Aufstreichen der Polymerlösung 2 auf die Oberfläche eines medizinischen Implantates;
d) Verdunsten des Lösungsmittels oder Lösungsmittelgemischs, sodass auf der Implantatoberfläche eine Polymerschicht zurückbleibt, in welcher das mindestens eine partikuläre Antiinfektivum dispergiert ist.
dadurch gekennzeichnet, dass die Polymerschicht keine Porenbildner aufweist.

Eine weitere besonders bevorzugte Ausführungsform des erfindungsgemäßen Verfahrens umfasst insbesondere die nachfolgenden Verfahrensschritte:
a) Auflösen von 9 Gew.-% bis 11 Gew.-% Gew.-% eines Polymethylmethacrylates oder eines Polymethyl-co-methylacrylates, welches durch eine zahlenmittlere Molmasse von größer als 200000 g/mol und eine Glasübergangstemperatur von größer 47 °C gekennzeichnet ist, in Essigsäureethylester oder einem Gemisch von Essigsäureethylester mit mindestens einem weiteren Lösungsmittel, welche durch einen Dampfdruck von mindestens 95 hPa bei 20 °C und Normaldruck gekennzeichnet sind, unter Bildung einer Polymerlösung 1;
b) Suspendieren von mindestens einem partikulären Antiinfektivum ausgewählt aus der Gruppe, bestehend aus Gentamicinsulfat, Vancomycinhydrochlorid, Clindamycinhydrochlorid, Daptomycin und Mischungen davon, in der Polymerlösung 1 unter Bildung einer Polymerlösung 2;
c) Aufstreichen der Polymerlösung 2 auf die Oberfläche eines medizinischen Implantates;
d) Verdunsten des Lösungsmittels oder Lösungsmittelgemischs, sodass auf der Implantatoberfläche eine Polymerschicht zurückbleibt, in welcher das mindestens eine partikuläre Antiinfektivum dispergiert ist, dadurch gekennzeichnet, dass die Polymerschicht keine Porenbildner aufweist.

Das erfindungsgemäße Verfahren führt zu beschichteten Gegenständen, die eine Polymerschicht aufweisen, in welcher ein pharmazeutischer Wirkstoff dispergiert ist. Im Anschluss kann der Gegenstand, beispielweise in der Form eines Implantats, anschließend in einen lebenden Organismus eingebracht werden, an welchem der Wirkstoff kontinuierlich freigesetzt werden soll.

Die vorliegende Erfindung umfasst auch ein Kit-of-parts und eine Vorrichtung, welche ausgehend von dem Kit-of-parts zur Durchführung des erfindungsgemäßen Verfahrens erhalten wird. Das erfindungsgemäße Kit-of-parts und die Vorrichtungen werden im Nachgang näher beschrieben.

### III. Kit-of-parts und Vorrichtung

Im Sinne der vorliegenden Erfindung wird auch ein Kit-of-parts (im Folgenden als Kit bezeichnet) zur Ausführung des erfindungsgemäßen Verfahrens beansprucht. Das Kit im Sinne der vorliegenden Erfindung stellt alle Bestandteile bereit, die Fachpersonal zur Ausführung des erfindungsgemäßen Verfahrens benötigt.

Daneben wird aber auch eine Vorrichtung beansprucht, die dazu dient, das erfindungsgemäße Verfahren auszuführen und ausgehend von dem erfindungsgemäßen Kit erhalten wird.

Das erfindungsgemäße Kit umfasst die folgenden Bestandteile:
i) ein lösemittelbeständiger Behälter, der eine Öffnung aufweist;
ii) ein lösemittelbeständiges Verschlusselement, welches zum Verschließen des Behälters geeignet ist;
iii) eine Auftragehilfe;
iv) eine Polymerlösung 1, wie zuvor beschrieben;
v) gegebenenfalls einen Mischkörper; und
vi) gegebenenfalls ein oder mehrere pharmazeutische Wirkstoffe in separaten Verpackungsmitteln.

Im Sinne der vorliegenden Erfindung wird für das Kit also zumindest ein lösemittelbeständiger Behälter bereitgestellt, der dazu geeignet ist, Lösungsmittel oder Lösungsmittelgemische, wie zuvor beschrieben, aufzunehmen, ohne dadurch beschädigt zu werden. Weiterhin wird mindestens ein Verschlusselement für den Behälter, beispielsweise ein Schraub- oder Steckverschluss, bereitgestellt, der ebenfalls lösungsmittelbeständig ist. Dadurch ist es möglich, eine gegebenenfalls vorbereitete Polymerlösung in dem geeigneten Behältnis nach Bedarf für Fachpersonal bereitzustellen.

Im Sinne der vorliegenden Erfindung wird das Kit auch mit einer Auftragehilfe ausgestattet, wozu jedes denkbare Element geeignet ist, um eine erfindungsgemäß bereitgestellte Lösung oder Suspension gleichmäßig auf einer Oberfläche zu verteilen.

Im Sinne der vorliegenden Erfindung ist es von Vorteil, wenn eine fertige Polymerlösung 1, wie zuvor beschrieben, bereits im lösemittelbeständigen Behälter vorgelegt wird. Soll beispielsweise ein Implantat kurz vor dessen Einsetzen *in-vivo* beschichtet werden, ist es für das Fachpersonal einfacher, wenn nicht zunächst benötigte Mengen von Polymer oder Copolymer eingewogen und in einem geeigneten Lösungsmittel gelöst werden müssen.

In einer bevorzugten Ausgestaltung umfasst das erfindungsgemäße Kit daher in dem lösemittelbeständigen Behälter bereits eine wie zuvor beschriebene Polymerlösung 1.

Im Sinne der vorliegenden Erfindung ist weiterhin denkbar, dass ein Mischkörper, wie zuvor beschrieben, dem Kit zur Ausführung des erfindungsgemäßen Verfahrens beigefügt wird. Prinzipiell kann ein pharmazeutischer Wirkstoff durch Schütteln des verschlossenen Behälters mit der Polymerlösung 1 ausreichend gelöst oder dispergiert werden. Um den Vorgang zu erleichtern, kann jedoch auch ein Mischkörper, wie ein Mischstab, dem Kit beigelegt werden, oder Mischkörper, wie Mischelemente, direkt im Behälter für die Polymerlösung 1 vorgesehen sein.

Es ist auch denkbar, das erfindungsgemäße Kit von vorneherein so zu konfigurieren, dass separat verpackte pharmazeutische Wirkstoffe bereitgestellt werden, um auf möglichst einfache Weise eine Polymerlösung 2 mit einer Sollkonzentration zu erhalten. Dies ist jedoch nicht zwingend erforderlich, da Flexibilität in der Wahl des pharmazeutischen Wirkstoffes oder in der Wahl von dessen Konzentration auch wünschenswert sein kann.

Mit dem erfindungsgemäßen Kit kann beispielsweise medizinisches Fachpersonal im OP-Saal innerhalb kürzester Zeit beliebige pharmazeutische Wirkstoffe zur Polymerlösung 1 zumischen und mit der Auftragehilfe unmittelbar nach dem Mischen die resultierende Polymerlösung 2 auch Oberflächen, beispielsweise von Implantaten, auftragen.

Im Sinne des erfindungsgemäßen Kits ist es bevorzugt, wenn der Behälter über eine Öffnung verfügt, welche einen Durchmesser von mindestens 1 cm, bevorzugt mindestens 2 cm, besonders bevorzugt mindestens 3 cm, aufweist. Beim Vermischen der Polymerlösung 1 mit einem geeigneten pharmazeutischen Wirkstoff ist es vorteilhaft, wenn die Öffnung eine der bevorzugten Mindestgrößen aufweist. Dadurch kann mit einem Mischkörper, wie einem Rührstab oder einem Spatel, durch die Öffnung des Behälters die Polymerlösung 1 erreicht und mit einem pharmazeutischen Wirkstoff vermischt werden.

Die Öffnung des Behälters ist vorzugsweise oben an dem Behälter vorgesehen.

Die erfindungsgemäßen Auftragehilfen können alle Arten von Gegenständen umfassen, mit denen ein Auftragen einer Lösung oder Suspension auf die Oberfläche eines Gegenstands ermöglicht wird. Im Sinne der vorliegenden Erfindung ist es bevorzugt, wenn dabei solche Auftragehilfen verwendet werden, die auf einfache Weise in dem Kit vorgesehen sein können, beispielsweise ein Pinsel oder eine Zerstäuberkappe. Erfindungsgemäß können diese Auftragehilfen beispielsweise mit dem lösemittelbeständigen Verschlusselement kombiniert werden.

Mischkörper im Sinne der erfindungsgemäßen Vorrichtung umfassen insbesondere die zuvor beschriebenen, für das erfindungsgemäße Verfahren geeigneten Mischkörper. Dies umfasst also einerseits beispielsweise Mischstäbe wie Rührstäbe oder Spatel. Alternativ ist es möglich, dass in dem Behälter mindestens ein freibeweglicher Mischkörper angeordnet ist, wobei Kunststoffkugeln, Glaskugeln und Keramikkugeln als Mischkörper bevorzugt sind. Bei dieser Ausgestaltungsvariante wird der pharmazeutische Wirkstoff zu der Polymerlösung 1 gegeben und das Gemisch nach Verschluss des Behälters geschüttelt. Durch Einwirkung des Mischkörpers werden die pharmazeutischen Wirkstoffe in der Polymerlösung 1 suspendiert oder gelöst.

Der Durchmesser der Mischkörper, insbesondere der Mischelemente, ist bevorzugt maximal halb so groß wie der Innendurchmesser des Behälters. Das bedeutet, dass beim Mischen die Polymerlösung noch genügend Raum neben dem Mischkörper hat, um bei seiner Bewegung im Behälter um den Mischkörper strömen zu können.

In einer bevorzugten Ausführungsvariante des erfindungsgemäßen Kits sind zumindest einige der Bestandteile des Kits physisch miteinander verbunden. Dies betrifft bevorzugt den Behälter und das Verschlusselement und/oder das Verschlusselement und die Auftragehilfe. Beispielsweise kann das Verschlusselement am Behälter befestigt sein, sodass es nicht versehentlich verlegt wird, wenn ein pharmazeutischer Wirkstoff in die Polymerlösung 1 gegeben werden soll. Es ist dabei ebenfalls bevorzugt, wenn eine Auftragehilfe, wie ein Pinsel, beispielsweise innseitig mit dem Verschlusselement verbunden ist, oder wenn das Verschlusselement beispielsweise selbst eine Zerstäuberkappe ist.

Eine besonders bevorzugte Variante des erfindungsgemäßen Kits umfasst daher insbesondere die folgenden Bestandteile:
i) ein lösemittelbeständiger Behälter, der eine Öffnung besitzt;
ii) ein lösemittelbeständiges Verschlusselement, welches zum Verschließen des Behälters geeignet ist und gegebenenfalls am Behälter fixiert ist;
iii) einen Pinsel, der gegebenenfalls innseitig im Verschlusselement fixiert ist, sodass er, wenn das Verschlusselement zum Verschließen des Behälters verwendet wird, innseitig in den Behälter ragt;
iv) einer Polymerlösung 1, wie zuvor beschrieben;
v) mindestens einen Mischkörper im Behälter oder einen separaten Mischstab; und
vi) gegebenenfalls ein oder mehrere pharmazeutische Wirkstoffe in separaten Verpackungsmitteln.

Ausgehend von diesem erfindungsgemäßen Kit kann vor der Durchführung des erfindungsgemäßen Verfahrens eine entsprechende Vorrichtung bereitgestellt werden. Entsprechende obige Ausführungen zu dem erfindungsgemäßen Kit gelten auch für die ebenfalls beanspruchte Vorrichtung.

### IV. Medizinisches Implantat und Prävention von Infektionen

Im Sinne der vorliegenden Erfindung ist es möglich, mit dem erfindungsgemäßen Verfahren einen Gegenstand, wie ein medizinisches Implantat mit einer pharmazeutisch aktiven Polymerbeschichtung auszustatten, welches dann zur Prävention von Infektionen anstatt eines unbeschichteten Implantats eingesetzt werden kann.

Die vorliegende Erfindung betrifft daher auch ein medizinisches Implantat, welches die oben beschriebene Beschichtung aufweist.

Ein erfindungsgemäßes medizinisches Implantat ist mit einer entsprechend des erfindungsgemäßen Verfahrens erhältlichen Polymerbeschichtung, in welcher ein pharmazeutischer Wirkstoff dispergiert ist, ausgestattet. Die Polymerbeschichtung ist damit durch einen Anteil von Polymer oder Copolymer im Verhältnis zum Anteil des pharmazeutischen Wirkstoffes zwischen 1,00 zu 0,05 bis 1,00 zu 1,50, wie zuvor beschreiben, gekennzeichnet.

Die Polymerbeschichtung des medizinischen Implantates umfasst ein Polymer mit einer Glasübergangstemperatur (Tg) von größer als 47 °C, bevorzugt größer als 60 °C, bevorzugter größer als 80 °C, insbesondere größer als 100 °C, und einer zahlenmittleren Molmasse (Mₙ) von größer als 200000 g/mol, weiter bevorzugt größer als 400000 g/mol, weiter bevorzugt größer als 600000 g/mol, wie zuvor beschrieben.

Die Polymerbeschichtung des medizinischen Implantates weist vorzugsweise keine Risse oder sichtbare Anzeichen von Sprödigkeit auf und ist durch eine Schichtdicke von 50 µm bis 200 µm, wie zuvor beschrieben, gekennzeichnet.

Ein beschichtetes medizinisches Implantat kann eine pharmazeutische Wirkung entfalten, wenn in der Polymerbeschichtung ein pharmazeutischer Wirkstoff dispergiert wurde. Erfindungsgemäß wurde festgestellt, dass der dispergierte Wirkstoff in einer warmen und wässrigen Umgebung aus der Matrix der Polymerbeschichtung abgegeben wird.

Nach einem operativen Eingriff ist das Risiko einer Infektion üblicherweise erhöht, da Knochen oder Gewebematerial beispielsweise durch Inzisionen der äußeren Umgebung ausgesetzt werden. Außerdem sind Patienten, die einer Operation unterzogen werden, häufig geschwächt, was ihre körpereigne Infektionsabwehr herabsetzt.

Im Sinne der vorliegenden Erfindung kann ein medizinisches Implantat, welches mit dem erfindungsgemäßen Verfahren mit einer pharmazeutisch aktiven Polymerbeschichtung ausgestattet wurde, insbesondere ein intramedullärer Nagel oder eine Osteosyntheseplatte sein, welche dann infektionspräventiv an der eingesetzten Stelle in-vivo wirken. Intramedulläre Nägel werden bei der Marknagelung verwendet, womit Brüche von Röhrenknochen gerichtet werden. Eine Osteosyntheseplatten wird in der Osteosynthese verwendet, bei welcher es sich um ein operatives Verfahren zur Behandlung von Knochenbrüchen handelt, wobei die Knochenbruchstücke mit Hilfe von dieser Platte verbunden werden.

Im Sinne der vorliegenden Erfindung kann ein medizinisches Implantat, welches mit dem erfindungsgemäßen Verfahren mit einer pharmazeutisch aktiven Polymerbeschichtung ausgestattet wurde, also insbesondere präventiv Infektionen nach einer Operation bei einem Knochenbruch, wie der Marknagelung, verhindern.

### BEISPIELE

### a) Untersuchungen zur Beschaffenheit von Polymerbeschichtungen

Es wurden Polymethylmethacrylate und Polymethylmethacrylat-co-methylacrylate mit den zahlenmittleren Molmassen von ~100000 g/mol, ~200000 g/mol, ~550000 g/mol, ~700000 g/mol., ~1250000 g/mol zu 5,0 Gew-% und zu 10.-Gew.-% in Essigsäureethylester gelöst. Diese Lösungen wurden auf eine Edelstahloberfläche gestrichen und die Beschaffenheit der verbliebenen Polymerschicht nach Abdunsten des Essigsäureethylesters geprüft. Es zeigte sich, dass mit Poylmethylmethacrylat mit einer zahlenmittleren Molmasse von kleiner 200.000 g/mol sprödere und schlechter haftende Beschichtungen erhalten wurden als mit Polymethylmethacrylat und Polymethylmethacrylat-co-methylacrylat mit einer zahlenmittleren Molmasse von größer/gleich 200000 g/mol.

### b) Untersuchungen der Wirkstoffkonzentration in Polymerbeschichtungen

Es wurden für die folgenden Versuche ein Polymethylmethacrylat-methylacrylat-Copolymer mit einer zahlenmittleren Molmasse von ~550000 g/ml und ein Edelstahlzylinder (CoCr28Mo6, ASTM F75-Legierung) mit einem Durchmesser von 10,0 mm und einer Länge von 60,0 mm verwendet. Gentamicinsulfat, Vancomycinhydrochlorid, Clindamycinhydrochlorid und Daptomycin wurden mit einer 10 gew.-%igen Lösung des Polymethylmethacrylat-methylacrylat-Copolymers in Essigsäureethylester vermischt (Tabellen 2 und 3). Die Antiinfektiva lagen als Pulver mit einer mittleren Partikelgröße kleiner 100 µm vor. Die gebildeten Dispersionen waren milchig-trüb.

Die Gemische 1 bis 10 wurden auf die Edelstahlzylinder durch Pinseln aufgetragen. Dabei wurde nur eine Hälfte des Stahlzylinders mit einer Fläche von 10,20 cm² beschichtet. Es wurden pro Gemisch jeweils 5 Edelstahlzylinder beschichtet. Die Masse der Beschichtung wurde nach Abdunstung des Essigsäureethylester gravimetrisch bestimmt. Anschließend wurden die beschichteten Edelstahlzylinder für 24 Stunden in dest. Wasser bei 37 °C gelagert. Danach wurden die Edelstahlzylinder getrocknet und der Masseverlust infolge der Freisetzung der Antiinfektiva gravimetrisch bestimmt.

**Tabelle 2. Zusammensetzungen der Gemische 1 bis 7; konstante Massen von Lösungsmittel und Copolymer, variierende Massen des pharmazeutischen Wirkstoffes.**

| **Gemisch-Nr.** | **Essigsäureethylester** | **Polymethlmethacrylat-co-methylacrylat** | **Gentamicinsulfat** |
|---|---|---|---|
| 1 | 10,00 g | 1,00 g | 0,25 g |
| 2 | 10,00 g | 1,00 g | 0,50 g |
| 3 | 10,00 g | 1,00 g | 0,75 g |
| 4 | 10,00 g | 1,00 g | 1,00 g |
| 5 | 10,00 g | 1,00 g | 1,25 g |
| 6 | 10,00 g | 1,00 g | 1,50 g |
| 7 | 10,00 g | 1,00 g | 2,50 g |

**Tabelle 3. Zusammensetzung der Gemische 8 bis 10; konstante Massen von Lösungsmittel und Copolymer, variierende pharmazeutische Wirkstoffe mit jeweils konstanter Masse.**

| **Gemisch-Nr.** | **Essigsäure-ethylester** | **Polymethl-methacrylat** | **Vancomycin-hydrochlorid** | **Clindamycin-hydrochlorid** | **Daptomycin** |
|---|---|---|---|---|---|
| 8 | 10,00 g | 1,00 g | 1,0 | - | - |
| 9 | 10,00 g | 1,00 g | - | 1,0 g | - |
| 10 | 10,00 g | 1,00 g | - | - | 1,0g |

Die Tabellen 2 und 3 geben an, wie die als Polymerlösung 2 bezeichnete Mischung für die Gemische 1 bis 10 jeweils zusammengesetzt war. Anhand der Gemische 1 bis 7 wurde der Einfluss einer steigenden Wirkstoffkonzentration untersucht. Anhand der Gemische 8 bis 10 wurde der Einfluss der Stoffidentität bei gleichem Massenanteil untersucht.

**Tabelle 4. Gravimetrische Untersuchungen an fünf Proben a bis e aus dem Gemisch 1; Masse der Polymerbeschichtung nach dem Auftragen und nach der Lagerung in 37 °C warmem Wasser.**

| **Beschichtung mit Gemisch Nr. 1** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0084 | 0,0053 | 0,0031 | 37 |
| b | 0,0107 | 0,0075 | 0,0032 | 30 |
| c | 0,0081 | 0,0057 | 0,0024 | 30 |
| d | 0,0078 | 0,0054 | 0,0024 | 31 |
| e | 0,0102 | 0,0066 | 0,0036 | 35 |
| | | | **Mittelwert des Masseverlustes** | 33 |

Der theoretische Masseverlust bei vollständigem Herauslösen des Gentamicinsulfat beträgt 20 Gew.-%. Es wurde ein mittlerer Masseverlust von 33 Gew.-% gefunden.

**Tabelle 5. Gravimetrische Untersuchungen an fünf Proben a bis e aus dem Gemisch 2; Masse der Polymerbeschichtung nach dem Auftragen und nach der Lagerung in 37 °C warmem Wasser.**

| **Beschichtung mit Gemisch Nr. 2** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0072 | 0,0038 | 0,0034 | 47 |
| b | 0,0065 | 0,0040 | 0,0025 | 38 |
| c | 0,0092 | 0,0052 | 0,0040 | 43 |
| d | 0,0111 | 0,0064 | 0,0047 | 42 |
| e | 0,0070 | 0,0041 | 0,0029 | 41 |
| | | | **Mittelwert des Masseverlustes** | 42 |

Der theoretische Masseverlust bei vollständigem Herauslösen des Gentamicinsulfat beträgt 33 Gew.-%. Es wurde ein mittlerer Masseverlust von 42 Gew.-% gefunden.

**Tabelle 6. Gravimetrische Untersuchungen an fünf Proben a bis e aus dem Gemisch 3; Masse der Polymerbeschichtung nach dem Auftragen und nach der Lagerung in 37 °C warmem Wasser.**

| **Beschichtung mit Gemisch Nr. 3** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0128 | 0,0070 | 0,0058 | 45 |
| b | 0,0113 | 0,0067 | 0,0046 | 41 |
| c | 0,0151 | 0,0092 | 0,0059 | 38 |
| d | 0,0150 | 0,0094 | 0,0056 | 37 |
| e | 0,0144 | 0,0086 | 0,0058 | 40 |
| | | | **Mittelwert des Masseverlustes** | 40 |

Der theoretische Masseverlust bei vollständigem Herauslösen des Gentamicinsulfat beträgt 43 Gew.-%. Es wurde ein mittlerer Masseverlust von 40 Gew.-% gefunden.

**Tabelle 7. Gravimetrische Untersuchungen an fünf Proben a bis e aus dem Gemisch 4; Masse der Polymerbeschichtung nach dem Auftragen und nach der Lagerung in 37 °C warmem Wasser.**

| **Beschichtung mit Gemisch Nr. 4** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0074 | 0,0036 | 0,0038 | 51 |
| b | 0,0075 | 0,0039 | 0,0036 | 48 |
| c | 0,0102 | 0,0052 | 0,0050 | 49 |
| d | 0,0134 | 0,0065 | 0,0069 | 51 |
| e | 0,0152 | 0,0073 | 0,0079 | 52 |
| | | | **Mittelwert des Masseverlustes** | 50 |

Der theoretische Masseverlust bei vollständigem Herauslösen des Gentamicinsulfat beträgt 50 Gew.-%. Es wurde ein mittlerer Masseverlust von 50 Gew.-% gefunden.

**Tabelle 8. Gravimetrische Untersuchungen an fünf Proben a bis e aus dem Gemisch 5; Masse der Polymerbeschichtung nach dem Auftragen und nach der Lagerung in 37 °C warmem Wasser.**

| **Beschichtung mit Gemisch Nr. 5** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0100 | 0,0044 | 0,0056 | 56 |
| b | 0,0104 | 0,0045 | 0,0059 | 57 |
| c | 0,0160 | 0,0066 | 0,0094 | 59 |
| d | 0,0146 | 0,0069 | 0,0077 | 53 |
| e | 0,0098 | 0,0046 | 0,0052 | 53 |
| | | | **Mittelwert des Masseverlustes** | 56 |

Der theoretische Masseverlust bei vollständigem Herauslösen des Gentamicinsulfats beträgt 55 Gew.-%. Es wurde ein mittlerer Masseverlust von 56 Gew.-% gefunden.

**Tabelle 9. Gravimetrische Untersuchungen an fünf Proben a bis e aus dem Gemisch 6; Masse der Polymerbeschichtung nach dem Auftragen und nach der Lagerung in 37 °C warmem Wasser.**

| **Beschichtung mit Gemisch Nr. 6** | **Masse der Beschichtung [g]** | **Masse der Beschichtung nach Lagerung in Wasser bei 37°C [g]** | **Masseverlust [mg]** | **Masseverlust [%]** |
|---|---|---|---|---|
| a | 0,0138 | 0,0052 | 0,0086 | 62 |
| b | 0,0161 | 0,0060 | 0,0101 | 63 |
| c | 0,0152 | 0,0056 | 0,0096 | 63 |
| d | 0,0142 | 0,0053 | 0,0089 | 63 |
| e | 0,0180 | 0,0063 | 0,0117 | 65 |
| | | | **Mittelwert des Masseverlustes** | 63 |

Der theoretische Masseverlust bei vollständigem Herauslösen des Gentamicinsulfat beträgt 60 Gew.-%. Es wurde ein mittlerer Masseverlust von 63 Gew.-% gefunden.

**Tabelle 10. Gravimetrische Untersuchungen an fünf Proben a bis e aus dem Gemisch 7; Masse der Polymerbeschichtung nach dem Auftragen und nach der Lagerung in 37 °C warmem Wasser.**

| **Beschichtung mit Gemisch Nr. 7** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0147 | 0,0043 | 0,0104 | 71 |
| b | 0,0207 | 0,0058 | 0,0149 | 72 |
| c | 0,0180 | 0,0053 | 0,0127 | 71 |
| d | 0,0152 | 0,0043 | 0,0109 | 72 |
| e | 0,0212 | 0,0065 | 0,0147 | 69 |
| | | | **Mittelwert des Masseverlustes** | 71 |

Der theoretische Masseverlust bei vollständigem Herauslösen des Gentamicinsulfat beträgt 71 Gew.-%. Es wurde ein mittlerer Masseverlust von 71 Gew.-% gefunden.

**Tabelle 11. Gravimetrische Untersuchungen an fünf Proben a bis e aus dem Gemisch 8; Masse der Polymerbeschichtung nach dem Auftragen und nach der Lagerung in 37 °C warmem Wasser.**

| **Beschichtung mit Gemisch Nr. 8** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0128 | 0,0065 | 0,0063 | 49 |
| b | 0,0112 | 0,0057 | 0,0055 | 49 |
| c | 0,0120 | 0,0060 | 0,0060 | 50 |
| d | 0,0104 | 0,0052 | 0,0052 | 50 |
| e | 0,0130 | 0,0066 | 0,0064 | 49 |
| | | | **Mittelwert des Masseverlustes** | 49 |

Der theoretische Masseverlust bei vollständigem Herauslösen des Vancomycinhydrochlorids beträgt 50 Gew.-%. Es wurde ein mittlerer Masseverlust von 49 Gew.-% gefunden. Das Vancomycinhydrochlorid wurde praktisch vollständig aus der Beschichtung herausgelöst.

**Tabelle 12. Gravimetrische Untersuchungen an fünf Proben a bis e aus dem Gemisch 9; Masse der Polymerbeschichtung nach dem Auftragen und nach der Lagerung in 37 °C warmem Wasser.**

| **Beschichtung mit Gemisch Nr. 9** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0079 | 0,0044 | 0,0035 | 44 |
| b | 0,0155 | 0,0080 | 0,0075 | 48 |
| c | 0,0118 | 0,0065 | 0,0053 | 45 |
| d | 0,0121 | 0,0066 | 0,0055 | 45 |
| e | 0,0082 | 0,0046 | 0,0036 | 44 |
| | | | **Mittelwert des Masseverlustes** | 45 |

Der theoretische Masseverlust bei vollständigem Herauslösen des Clindamycinhydrochlorids beträgt 50 Gew.-%. Es wurde ein Masseverlust von 45 Gew.-% gefunden. Das Clindamycinhydrochlorid wurde fast vollständig aus der Beschichtung herausgelöst.

**Tabelle 13. Gravimetrische Untersuchungen an fünf Proben a bis e aus dem Gemisch 10; Masse der Polymerbeschichtung nach dem Auftragen und nach der Lagerung in 37 °C warmem Wasser.**

| **Beschichtung mit Gemisch Nr. 10** | **Masse der Beschichtung [mg]** | **Masse der Beschichtung nach Lagerung in Wasser bei 37°C [mg]** | **Masseverlust [mg]** | **Masseverlust [%]** |
|---|---|---|---|---|
| a | 0,0121 | 0,0071 | 0,0050 | 41 |
| b | 0,0134 | 0,0078 | 0,0056 | 42 |
| c | 0,0050 | 0,0031 | 0,0019 | 38 |
| d | 0,0091 | 0,0054 | 0,0037 | 40 |
| e | 0,0106 | 0,0063 | 0,0043 | 40 |
| | | | **Mittelwert des Masseverlustes** | 40 |

Der theoretische Masseverlust bei vollständigem Herauslösen des Daptomycins beträgt 50 Gew.-%. Es wurde ein mittlerer Masseverlust von 40 Gew.-% gefunden. In der Beschichtung verblieb noch ein Rest an Daptomycin.

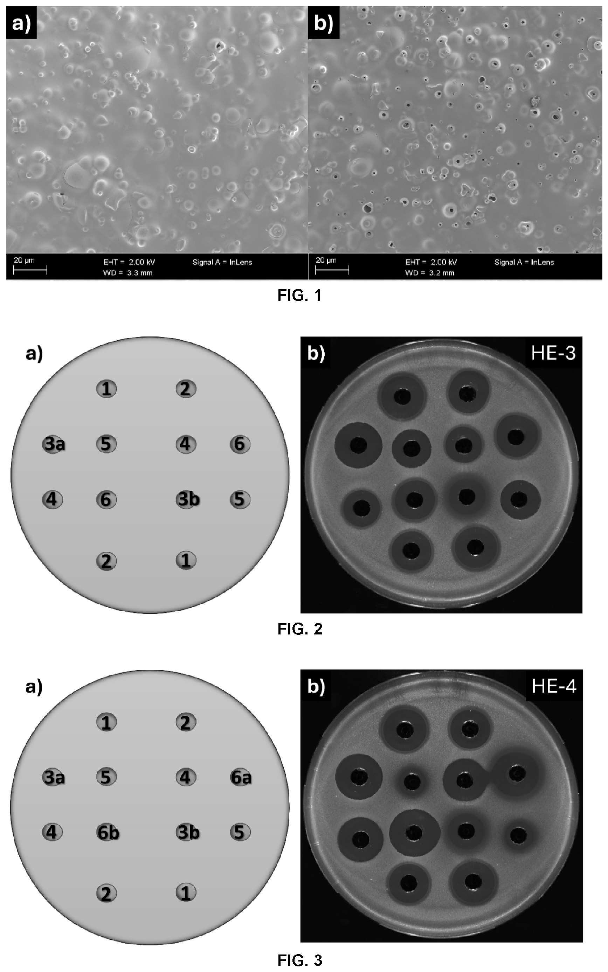

Es wurde untersucht, warum für die in den Tabellen 4, 5, 8 und 9 dargestellten Ergebnisse höhere mittlere Masseverluste erzielt wurden als theoretisch berechnet. Dazu wurde je eine Beschichtung vor der Lagerung in warmem Wasser und nach der Lagerung mit einem Rasterelektronenmikroskop untersucht (Figur 1).

Dabei wurde festgestellt, dass sich in der Polymerbeschichtung verteilte Verkapselungen oder Einschlüsse bilden, die sich bei der Lagerung im Wasser bei 37 °C öffnen. Es wird davon ausgegangen, dass sich kleine Partikel von der Polymerschicht ablösen oder aber durch Wägefehler es zu einem Masseverlust kommt.

Die Ergebnisse zeigen, dass eine annähernd quantitative Freisetzung der Antibiotika, im Rahmen des Wägefehlers, aus den Beschichtungen durch Einwirkung von Wasser erfolgte.

### c) Antimikrobielle Aktivität der Beschichtungen 4, 6, 8 und 10

Es wurden die Eluate der Versuche 4, 6, 8 und 10 auf ihre antimikrobielle Aktivität geprüft. Dazu wurden Hemmhoftests unter Verwendung des Testkeims *Bacillus subtilis* ATCC 6633 und des Nähragars 1 durchgeführt. Es wurde erhitzter Nähragar I mit einer Sporensuspension des *Bacillus subtilis* vermischt und der so beimpfte Nähragar I wurde in Petrischalen gegossen. Nach Abkühlung auf Raumtemperatur wurden Löcher in den erstarrten Agar gestanzt. Es wurden jeweils 50 µl unverdünntes Eluat oder 50 µl 1:50 verdünntes Eluat in ausgestanzte Löcher in den Nähragar eingebracht. Weiterhin wurden als Kontrollen wässrige Gentamicinsulfat-Lösung, Vancomycinhydrochlorid-Lösung und Clindamycinhydrochlorid-Lösung mitgeführt. Die Agarplatten wurden anschließend für 24 Stunden bei 37 °C inkubiert. Die Figuren 2 und 3 zeigen jeweils eine schematische Verteilung der unterschiedlichen Proben (bezeichnet als HE-3 und HE-4) auf der Platte sowie eine tatsächliche Bildaufnahme der Platte am Ende des Experiments.

**Tabelle 14. Beschreibung der unterschiedlichen Proben, die auf die Agarplatte aufgetragen wurden; korrespondierend zu Figur 2.**

| **Plattenname** | **Stanzloch** | **Probe** | **Verdünnung mit Wasser** |
|---|---|---|---|
| | 1 | Referenzlösung Gentamicinsulfat (Konzentration 20 µg/ml Genta-Base) | - |
| HE-3 | 2 | Referenzlösung Gentamicinsulfat (Konzentration 10 µg/ml Genta-Base) | - |
| | 3a | Referenzlösung Vancomycinhydrochlorid (Konzentration 5 µg/ml Vancomycin-base) | - |
| | 4 | Gemisch 4 | 1:50 |
| | 5 | Gemisch 8 | 1:50 |
| | 6 | Gemisch 6 | 1:50 |
| | 3b | Referenzlösung Clindamycinhydrochlorid (Konzentration 2 µg/ml Clindamycinbase) | - |

**Tabelle 15. Beschreibung der unterschiedlichen Proben, die auf die Agarplatte aufgetragen wurden; korrespondierend zu Figur 3.**

| **Plattenname** | **Stanzloch** | **Probe** | **Verdünnung** |
|---|---|---|---|
| HE-4 | 1 | Referenzlösung Gentamicinsulfat (Konzentration 20 µg/ml Genta-Base) | - |
| | 2 | Referenzlösung Gentamicinsulfat (Konzentration 10 µg/ml Genta-Base) | - |
| | 3a | Referenzlösung Vancomycinhydrochlorid (Konzentration 5 µg/ml Vancomycinbase) | - |
| | 4 | Gemisch 10 | 1:50 |
| | 5 | Gemisch 8 | 1:50 |
| | 6a | Referenzlösung Clindamycinhydrochlorid (Konzentration 4 µg/ml Clindamycinbase) | 1:50 |
| | 3b | Referenzlösung Clindamycinhydrochlorid (Konzentration 2µg/ml Clindamycinbase) | - |
| | 6b | Referenzlösung Vancomycinhydrochlorid (Konzentration 10 µg/ml Vancomycinbase) | |

### FIGURENBESCHREIBUNG

- **FIG. 1**: Rasterelektronenmikroskopaufnahmen einer erfindungsgemäß hergestellten Beschichtung a) vor der Inkubation des Trägers für 24 h bei 37 °C und b) Aufnahmen derselben Beschichtung nach dem Inkubationsvorgang. Unter a) ist eine Beschichtung abgebildet, in der Einschlüsse von Wirkstoff und gegebenenfalls nicht polymerisiertem Material in Form von geschlossenen Bläschen ersichtlich sind. Unter b) ist dieselbe Beschichtung abgebildet, jedoch wurden die Einschlüsse geöffnet, sichtbar anhand der dunkel gefärbten Öffnungen.
- **FIG. 2**: Bakterienbeimpfte Agarplatte HE-3, schematische Probenverteilung a) und Bildaufnahme des Trägers nach Inkubation für 24 h bei 37 °C b); Die in Tabelle 14 dargestellten Probenbeschreibungen korrespondieren zu dem unter a) dargestellten Schema. An den unter b) als dunkle Kreise sichtbaren Stanzlöchern ist jeweils eine der in Tabelle 14 dargestellten Probelösungen aufgetragen worden, die anschließend kreisrund in die Bakterienkultur diffundiert ist. Die Hemmhöfe, in denen die Bakterielle Konzentration abgenommen hat, sind als kreisrunde Bereiche um die Kavitäten sichtbar.
- **FIG. 3**: Bakterienbeimpfte Agarplatte HE-4, schematische Probenverteilung a) und Bildaufnahme des Trägers nach Inkubation für 24 h bei 37 °C b); Die in Tabelle 15 dargestellten Probenbeschreibungen korrespondieren zu dem unter a) dargestellten Schema. An den unter b) als dunkle Kreise sichtbaren Stanzlöchern ist jeweils eine der in Tabelle 15 dargestellten Probelösungen aufgetragen worden, die anschließend kreisrund in die Bakterienkultur diffundiert ist. Die Hemmhöfe, in denen die Bakterielle Konzentration abgenommen hat, sind als kreisrunde Bereiche um die Kavitäten sichtbar.

## Patentansprüche

1. Ein Verfahren zur Herstellung einer pharmazeutisch aktiven Polymerbeschichtung, umfassend die nachfolgenden Verfahrensschritte:
a) Auflösen eines nicht hydrolysierbares Polymers oder Copolymers, welches durch eine zahlenmittlere Molmasse von größer als 200000 g/mol, weiter bevorzugt eine zahlenmittlere Molmasse von größer als 400000 g/mol, weiter bevorzugt eine zahlenmittlere Molmasse von größer als 600000 g/mol, bestimmt mittels Gelpermeations-Chromatografie gekennzeichnet ist, in einem Lösungsmittel oder Lösungsmittelgemisch, welches durch einen Dampfdruck von mindestens 95 hPa bei 20 °C und Normaldruck gekennzeichnet ist, unter Bildung einer Polymerlösung 1;
b) Auflösen oder Suspendieren von mindestens einem pharmazeutischen Wirkstoff in der Polymerlösung 1 unter Bildung einer Polymerlösung 2;
c) Auftragen der Polymerlösung 2 auf die Oberfläche eines zu beschichtenden Gegenstandes; und
d) Verdunsten des Lösungsmittels oder Lösungsmittelgemischs, sodass auf der Oberfläche des zu beschichtenden Gegenstandes eine Polymerschicht zurückbleibt, in welcher der mindestens eine pharmazeutische Wirkstoff dispergiert ist,
**dadurch gekennzeichnet, dass** die Polymerschicht keine Porenbildner aufweist.

2. Verfahren gemäß Anspruch 1, **dadurch gekennzeichnet, dass** die Polymere oder Copolymere eine Glasübergangstemperatur von größer 47 °C, weiter bevorzugt größer als 60 °C, weiter bevorzugt größer als 80 °C, weiter bevorzugt größer als 100 °C, aufweisen.

3. Verfahren nach Anspruch 1 oder 2, **dadurch gekennzeichnet, dass** das Polymer oder Copolymer auf Polymethylmethacrylaten basiert und im Falle von Copolymeren das Comonomer ausgewählt ist aus der Gruppe, bestehend aus Methylacrylat, Ethylacrylat, Ethylmethacrylat, Styren und Mischungen davon.

4. Verfahren nach einem der Ansprüche 1 bis 3, **dadurch gekennzeichnet, dass** das Lösungsmittel oder Lösungsmittelgemisch einen Wassergehalt von kleiner als 8 Gew.-%, weiter bevorzugt von kleiner als 5 Gew.-% weiter bevorzugt von kleiner als 2 Gew.-%, aufweist.

5. Verfahren nach einem der Ansprüche 1 bis 4, **dadurch gekennzeichnet, dass** das Lösungsmittel oder Lösungsmittelgemisch ausgewählt ist aus der Gruppe, bestehend aus Essigsäureestern, Acetessigsäureestern, Aceton, Diacetonalkohol, Ethylmethylketon, Butylmethylketon und Mischungen davon.

6. Verfahren nach einem der Ansprüche 1 bis 5, **dadurch gekennzeichnet, dass** der Polymer- oder Copolymergehalt in der Polymerlösung 1 zwischen 2 Gew.-% und 18 Gew.- %, bevorzugt zwischen 9 Gew.-% und 11 Gew.-%, liegt.

7. Verfahren nach einem der Ansprüche 1 bis 6, **dadurch gekennzeichnet, dass** der pharmazeutische Wirkstoff ausgewählt ist aus der Gruppe, bestehend aus Antiinfektiva, Bakteriophagen, Cytostatika, Analgetika, Hormonen, Wachstumsfaktoren und Mischungen davon.

8. Verfahren nach einem der Ansprüche 1 bis 7, **dadurch gekennzeichnet, dass** der pharmazeutische Wirkstoff im Schritt b) als partikulärer Feststoff mit einer Partikelgröße kleiner als 250 µm, weiter bevorzugt mit einer Partikelgröße kleiner als 100 µm, bestimmt mittels einer Siebfraktionierung, zum Auflösen oder Suspendieren in die Polymerlösung 1 unter Erhalt einer Polymerlösung 2 gegeben wird.

9. Verfahren nach einem der Ansprüche 1 bis 8, **dadurch gekennzeichnet, dass** das Gewichtsverhältnis zwischen Polymeren oder Copolymeren und pharmazeutischen Wirkstoffen 1,00 zu 0,05 bis 1,00 zu 1,50 beträgt.

10. Verfahren nach einem der Ansprüche 1 bis 9, **dadurch gekennzeichnet, dass** die Polymerschicht eine Schichtdicke von 50 bis 500 µm aufweist.

11. Ein medizinisches Kit, insbesondere zur Ausführung des Verfahrens gemäß einem der Ansprüche 1 bis 10, umfassend die nachfolgenden folgenden Bestandteile:
i) ein lösemittelbeständiger Behälter, der eine Öffnung besitzt;
ii) ein lösemittelbeständiges Verschlusselement, welches zum Verschließen des Behälters geeignet ist;
iii) eine Auftragehilfe;
iv) eine Polymerlösung 1 erhältlich gemäß dem Verfahrens nach einem der Ansprüche 1 bis 10;
v) gegebenenfalls ein oder mehrere Mischkörper; und
vi) gegebenenfalls ein oder mehrere pharmazeutische Wirkstoffe in separaten Verpackungsmitteln.

12. Medizinisches Kit nach Anspruch 11, **dadurch gekennzeichnet, dass**
• die Öffnung des Behälters einen Durchmesser von mindestens 1 cm, bevorzugt mindestens 2 cm, weiter bevorzugt mindestens 3 cm aufweist, und/oder
• Auftragehilfen aus Pinseln oder Zerstäuberkappen ausgewählt sind, und/oder
• der Mischkörper aus mindestens einen Mischstab und/oder mindestens einem im Behälter in der Polymerlösung 1 vorgesehenen Mischelement ausgewählt ist, und/oder
• dass eines oder mehrere der unter Bestandteil i) bis iii) des medizinischen Kits aufgelisteten Elemente aneinander fixiert sind.

13. Eine Vorrichtung zur Ausführung des Verfahrens gemäß Anspruch 1 bis 10, umfassend die Bestandteile des medizinischen Kits gemäß Anspruch 11 oder 12.

14. Ein medizinisches Implantat, welches mit einer Polymerbeschichtung mit dispergiertem pharmazeutischen Wirkstoff beschichtet ist, erhältlich durch ein Verfahren gemäß Anspruch 1 bis 10.

15. Ein medizinisches Implantat gemäß Anspruch 14 zur Prävention von Infektionen nach einem operativen Eingriff.
